# EUROPEAN PATENT APPLICATION

(11) **EP 4 607 533 A1**
(43) Date of publication of application: **27.08.2025**
(21) Application number: 23890605.1
(22) Date of filing: 01.11.2023
(51) Int. Cl.: G16H 50/30

(54) **HEALTH MANAGEMENT METHOD, APPARATUS AND SYSTEM, ELECTRONIC DEVICE, AND STORAGE MEDIUM**

(30) Priority: 14.11.2022 CN 202211426125
(71) Applicant: Huawei Technologies Co., Ltd., Shenzhen, Guangdong 518129 (CN)
(72) Inventor: TENG, Teng, Shenzhen, Guangdong 518129 (CN); ZANG, Zhenfei, Shenzhen, Guangdong 518129 (CN)
(74) Representative: Gill Jennings & Every LLP
(86) International application number: PCT/CN2023/129022
(87) International publication number: WO 2024/104169

(57) **Abstract**

Embodiments of this application provide a health management method and apparatus, and a system, an electronic device, and a storage medium. The method includes: determining a target physiological indicator of a user; obtaining living data information and physiological data information of the user, and inputting the living data information and the physiological data information of the user into a prediction model, to obtain a prediction result output by the prediction model, where the prediction result represents a predicted impact of the living data information and the physiological data information of the user on a change of the target physiological indicator. The user may learn, based on the prediction result, whether the living data information and the physiological data information of the user can improve the target physiological indicator, thereby providing a possibility of long-term automatic and intelligent physical health detection for the user.

## Description

This application claims priority to Chinese Patent Application No. 202211426125.1, filed with the China National Intellectual Property Administration on November 14, 2022 and entitled "HEALTH MANAGEMENT METHOD AND APPARATUS, AND SYSTEM, ELECTRONIC DEVICE, AND STORAGE MEDIUM", which is incorporated herein by reference in its entirety.

### TECHNICAL FIELD

This application relates to the field of terminal device technologies, and specifically, to a health management method and apparatus, and a system, an electronic device, and a storage medium.

### BACKGROUND

With the improvement of modern living standards, people's awareness of health and healthcare is also improved, and more attention is paid to personal health management. Regular health examinations have become an important part of people's daily life. An existing physical examination report generally records physical examination data corresponding to a physical examination item and a normal range of the physical examination data, for example, a blood pressure value and a normal blood pressure range. A person undergoing physical examination learns a physical examination result of the person by viewing a physical examination report.

If there is an abnormal indicator item in the physical examination report, the user usually adjusts a living habit, a diet, and the like, to improve the abnormal indicator item. However, whether an adjusted living habit, an adjusted diet, and the like can improve the abnormal indicator can be learned only after the user goes to a hospital again for check.

### SUMMARY

In view of this, this application provides a health management method and apparatus, and a system, an electronic device, and a storage medium, to help resolve a conventional-technology problem that an electronic device cannot evaluate, based on living information of a user, whether an abnormal indicator is improved.

According to a first aspect, an embodiment of this application provides a health management method, applied to a first device. The method includes:
determining a target physiological indicator of a user; and
obtaining living data information and physiological data information of the user, and inputting the living data information and the physiological data information of the user into a prediction model, to obtain a prediction result output by the prediction model, where the prediction result represents a predicted impact of the living data information and the physiological data information of the user on a change of the target physiological indicator.

In a possible implementation of the first aspect, the method further includes:
determining, based on the target physiological indicator, a target reference user from a user whose physiological indicator data information, living data information, and physiological data information are obtained, where obtained physiological indicator data information of the target reference user includes data information of the target physiological indicator; and
constructing the prediction model based on the obtained physiological indicator data information, obtained living data information, and obtained physiological data information of the target reference user.

In a possible implementation of the first aspect, the determining a target physiological indicator of a user includes:
receiving the target physiological indicator of the user sent by a second device; and
the obtaining living data information and physiological data information of the user includes:
   receiving the living data information and the physiological data information of the user sent by the second device.

In a possible implementation of the first aspect, the method further includes:
receiving physiological indicator data information in a physical examination report of the user sent by the second device;
obtaining physiological indicator reference information of the user, and determining abnormal physiological indicator information and normal physiological indicator information of the user based on the physiological indicator reference information of the user and the physiological indicator data information of the user; and
sending the abnormal physiological indicator information and the normal physiological indicator information of the user to the second device.

In a possible implementation of the first aspect, the method further includes:
determining, based on the abnormal physiological indicator information of the user, a reference user for the user from another user whose physiological indicator data information is obtained; and
the determining, based on the target physiological indicator, a target reference user from a user whose physiological indicator data information, living data information, and physiological data information are obtained includes:
   determining, based on the target physiological indicator, the target reference user from a user that is in the reference user and whose physiological indicator data information, living data information, and physiological data information are obtained.

In a possible implementation of the first aspect, the method further includes:
determining a positive reference user from the target reference user, where the positive reference user is a user whose target physiological indicator is improved; and
generating improvement suggestion information for the target physiological indicator based on living data information and physiological data information of the positive reference user.

In a possible implementation of the first aspect, the determining a target physiological indicator of a user includes:
determining the target physiological indicator of the user in response to a selection operation of the user.

In a possible implementation of the first aspect, the method further includes:
sending the target physiological indicator to a third device; and
receiving the prediction model that is for the target physiological indicator and that is sent by the third device.

In a possible implementation of the first aspect, the obtaining living data information of the user includes:
obtaining the living data information of the user in response to an input operation of the living data information of the user.

In a possible implementation of the first aspect, the obtaining physiological data information of the user includes:
obtaining, from a fourth device, the physiological data information of the user detected by the fourth device.

In a possible implementation of the first aspect, the method further includes:
obtaining abnormal physiological indicator information and normal physiological indicator information of the user in response to a physical examination information input operation of the user; and
outputting the abnormal physiological indicator information and the normal physiological indicator information of the user.

In a possible implementation of the first aspect, the method further includes:
detecting whether the user has a physiological data detection device, where the physiological data detection device is a device for detecting at least one physiological indicator of the user; and
when it is detected that the user has the physiological data detection device, displaying the abnormal physiological indicator information in a preset sequence; and
the determining a target physiological indicator of a user in response to a selection operation of the user includes:
   determining the target physiological indicator of the user in response to the selection operation of the user on the abnormal physiological indicator information in the abnormal physiological indicator information displayed in the preset sequence.

In a possible implementation of the first aspect, the method further includes:
when it is detected that the user does not have the physiological data detection device, outputting information about a physiological data detection device capable of detecting an abnormal physiological indicator of the user.

In a possible implementation of the first aspect, the method further includes:
detecting whether the physiological data detection device of the user includes a fourth device, where the fourth device is a physiological data detection device capable of detecting the target physiological indicator of the user; and
the obtaining the prediction model for the target physiological indicator based on the target physiological indicator includes:
   when the physiological data detection device of the user includes the fourth device, obtaining the prediction model for the target physiological indicator based on the target physiological indicator.

In a possible implementation of the first aspect, the method further includes:
when it is detected that the physiological data detection device of the user does not include the fourth device, outputting information about the fourth device.

In a possible implementation of the first aspect, before the detecting whether the user has a physiological data detection device, the method further includes:
generating recommendation information based on the abnormal physiological indicator information of the user and displaying the recommendation information, where the recommendation information includes the information about the physiological data detection device capable of detecting the abnormal physiological indicator of the user and medical research information related to the abnormal physiological indicator of the user; and
the detecting whether the user has a physiological data detection device includes:
   in response to a first operation of the user on the recommendation information, detecting whether the user has the physiological data detection device.

In a possible implementation of the first aspect, the method further includes:
outputting information about the abnormal physiological indicator of the user in response to a second operation of the user on the recommendation information; or
in response to a third operation of the user on the recommendation information, outputting the information about the physiological data detection device capable of detecting the abnormal physiological indicator of the user; or
outputting the medical research information related to the abnormal physiological indicator of the user in response to a fourth operation of the user on the recommendation information.

In a possible implementation of the first aspect, the method further includes:
in response to a display operation of the user for medical research information, determining a display priority of each piece of the medical research information, and outputting a plurality of pieces of the medical research information based on display priorities, where the medical research information related to the abnormal physiological indicator of the user has a highest display priority.

In a possible implementation of the first aspect, the method further includes:
receiving improvement suggestion information sent by the third device; and
outputting the prediction result and the improvement suggestion information.

According to a second aspect, an embodiment of this application provides a health management method, applied to a second device. The method includes:
determining a target physiological indicator of a user in response to a selection operation of the user;
sending the target physiological indicator of the user to a first device; and
obtaining living data information and physiological data information of the user, and sending the living data information and the physiological data information of the user to the first device.

In a possible implementation of the second aspect, the obtaining living data information of the user includes:
obtaining the living data information of the user in response to an input operation of the living data information of the user.

In a possible implementation of the second aspect, the obtaining physiological data information of the user includes:
obtaining, from a fourth device, the physiological data information of the user monitored by the fourth device.

In a possible implementation of the second aspect, the method further includes:
in response to a physical examination information input operation of the user, performing recognition processing on physical examination information input by the user, to obtain physiological indicator data information of the user;
sending the physiological indicator data information of the user to the first device;
receiving abnormal physiological indicator information and normal physiological indicator information of the user sent by the first device; and
outputting the abnormal physiological indicator information and the normal physiological indicator information of the user.

In a possible implementation of the second aspect, the method further includes:
detecting whether the user has a physiological data detection device, where the physiological data detection device is a device for detecting at least one physiological indicator of the user; and
when it is detected that the user has the physiological data detection device, displaying the abnormal physiological indicator information in a preset sequence; and
the determining a target physiological indicator of a user in response to a selection operation of the user includes:
   determining the target physiological indicator of the user in response to the selection operation of the user on the abnormal physiological indicator information in the abnormal physiological indicator information displayed in the preset sequence.

In a possible implementation of the second aspect, the method further includes:
when it is detected that the user does not have the physiological data detection device, outputting information about a physiological data detection device capable of detecting an abnormal physiological indicator of the user.

In a possible implementation of the second aspect, the method further includes:
detecting whether the physiological data detection device of the user includes a fourth device, where the fourth device is a physiological data detection device capable of detecting the target physiological indicator of the user; and
the sending the target physiological indicator of the user to a first device includes:
   when the physiological data detection device of the user includes the fourth device, sending the target physiological indicator of the user to the first device.

In a possible implementation of the second aspect, the method further includes:
when it is detected that the physiological data detection device of the user does not include the fourth device, outputting information about the fourth device.

In a possible implementation of the second aspect, before the detecting whether the user has a physiological data detection device, the method further includes:
generating recommendation information based on the abnormal physiological indicator information of the user and displaying the recommendation information, where the recommendation information includes at least one of the information about the physiological data detection device capable of detecting the abnormal physiological indicator of the user and medical research information related to the abnormal physiological indicator of the user; and
the detecting whether the user has a physiological data detection device includes:
   in response to a first operation of the user on the recommendation information, detecting whether the user has the physiological data detection device.

In a possible implementation of the second aspect, the method further includes:
outputting information about the abnormal physiological indicator of the user in response to a second operation of the user on the recommendation information; or
in response to a third operation of the user on the recommendation information, outputting the information about the physiological data detection device capable of detecting the abnormal physiological indicator of the user; or
outputting the medical research information related to the abnormal physiological indicator of the user in response to a fourth operation of the user on the recommendation information.

In a possible implementation of the second aspect, the method further includes:
in response to a display operation of the user for medical research information, determining a display priority of each piece of the medical research information, and outputting a plurality of pieces of the medical research information based on display priorities, where the medical research information related to the abnormal physiological indicator of the user has a highest display priority.

In a possible implementation of the second aspect, the method further includes:
receiving improvement suggestion information sent by the first device; and
outputting the prediction result and the improvement suggestion information.

According to a third aspect, an embodiment of this application provides a health management apparatus, including:
an obtaining unit, configured to determine a target physiological indicator of a user; and
a processing unit, configured to obtain living data information and physiological data information of the user, and input the living data information and the physiological data information of the user into a prediction model, to obtain a prediction result output by the prediction model, where the prediction result represents a predicted impact of the living data information and the physiological data information of the user on a change of the target physiological indicator.

According to a fourth aspect, an embodiment of this application provides a health management apparatus, including:
an obtaining unit, configured to determine a target physiological indicator of a user in response to a selection operation of the user; and
a sending unit, configured to send the target physiological indicator of the user to a first device.

The obtaining unit is further configured to obtain living data information and physiological data information of the user.

The sending unit is further configured to send the living data information and the physiological data information of the user to the first device.

According to a fifth aspect, an embodiment of this application provides an electronic device, including a memory configured to store computer program instructions and a processor configured to execute the program instructions. When the computer program instructions are executed by the processor, the electronic device is triggered to perform the method according to any one of the implementations of the first aspect or the method according to any one of the implementation of the second aspect.

According to a sixth aspect, an embodiment of this application provides a computer-readable storage medium. The computer-readable storage medium includes a stored program, and when the program is run, a device in which the computer-readable storage medium is located is controlled to perform the method according to any one of the implementation of the first aspect or the method according to any one of the implementation of the second aspect.

According to solutions provided in embodiments of this application, the target physiological indicator of the user is determined, and the prediction model for the target physiological indicator is obtained based on the target physiological indicator. The living data information and the physiological data information of the user are obtained, and the living data information and the physiological data information of the user are input into the prediction model, to obtain the prediction result output by the prediction model. The prediction result is a prediction of an impact of the living data information and the physiological data information of the user on the change trend of the target physiological indicator. In this way, in embodiments of this application, after the living data information and the physiological data information of the user are obtained, the living data information and the physiological data information may be input into the prediction model for the target physiological indicator. The prediction model may perform prediction based on the living data information and the physiological data information of the user, to obtain the prediction result. The user may learn, based on the prediction result, whether the living data information and the physiological data information of the user can improve the target physiological indicator, thereby providing a possibility of long-term automatic and intelligent physical health detection for the user, and guiding the user to healthy exercise and diet, to get rid of a potential deterioration trend, and guide optimization of the target physiological indicator.

### BRIEF DESCRIPTION OF DRAWINGS

To describe technical solutions in embodiments of this application more clearly, the following briefly describes the accompanying drawings for embodiments. It is clear that the accompanying drawings in the following descriptions show merely some embodiments of this application, and a person of ordinary skill in the art may derive other drawings from these accompanying drawings without creative efforts.
FIG. 1a is a schematic flowchart of a health management method according to an embodiment of this application;
FIG. 1b is a schematic flowchart of another health management method according to an embodiment of this application;
FIG. 2 is a diagram of a health management scenario according to an embodiment of this application;
FIG. 3 is a diagram of another health management scenario according to an embodiment of this application;
FIG. 4a is a schematic flowchart of another health management method according to an embodiment of this application;
FIG. 4b is a schematic flowchart of another health management method according to an embodiment of this application;
FIG. 5A to FIG. 5D are a schematic flowchart of another health management method according to an embodiment of this application;
FIG. 6A and FIG. 6B are a diagram of another health management scenario according to an embodiment of this application;
FIG. 7a is a diagram of another health management scenario according to an embodiment of this application;
FIG. 7b is a diagram of another health management scenario according to an embodiment of this application;
FIG. 7c is a diagram of another health management scenario according to an embodiment of this application;
FIG. 7d is a diagram of another health management scenario according to an embodiment of this application;
FIG. 8 is a diagram of another health management scenario according to an embodiment of this application;
FIG. 9 is a diagram of another health management scenario according to an embodiment of this application;
FIG. 10A and FIG. 10B are a diagram of another health management scenario according to an embodiment of this application;
FIG. 11A and FIG. 11B are a diagram of another health management scenario according to an embodiment of this application;

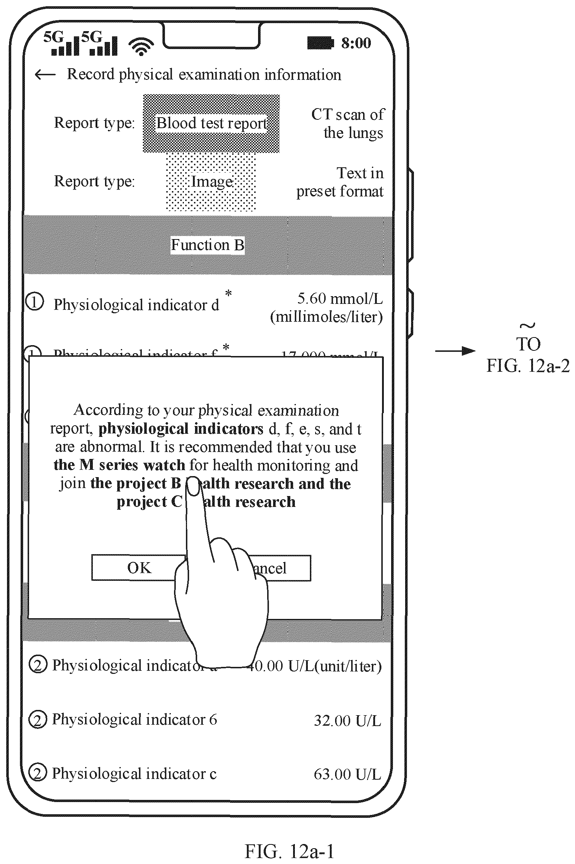

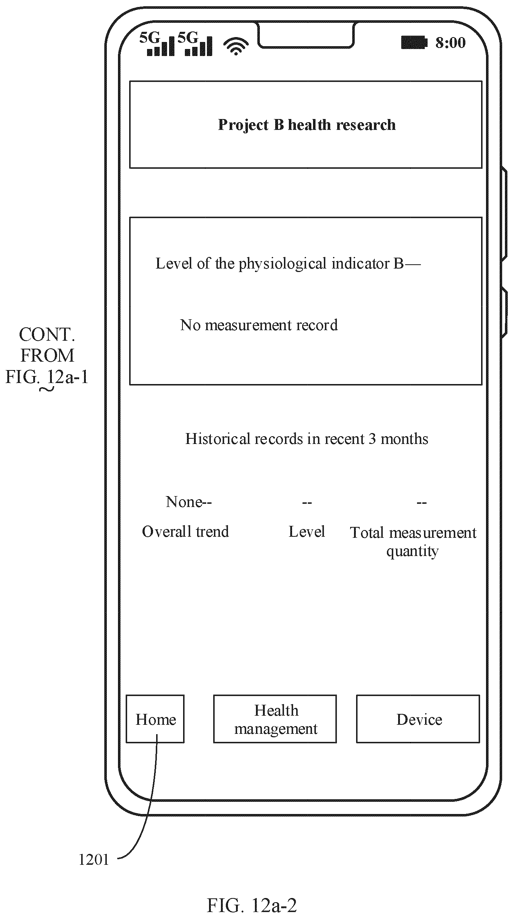

FIG. 12a-1 and FIG. 12a-2 are a diagram of another health management scenario according to an embodiment of this application;

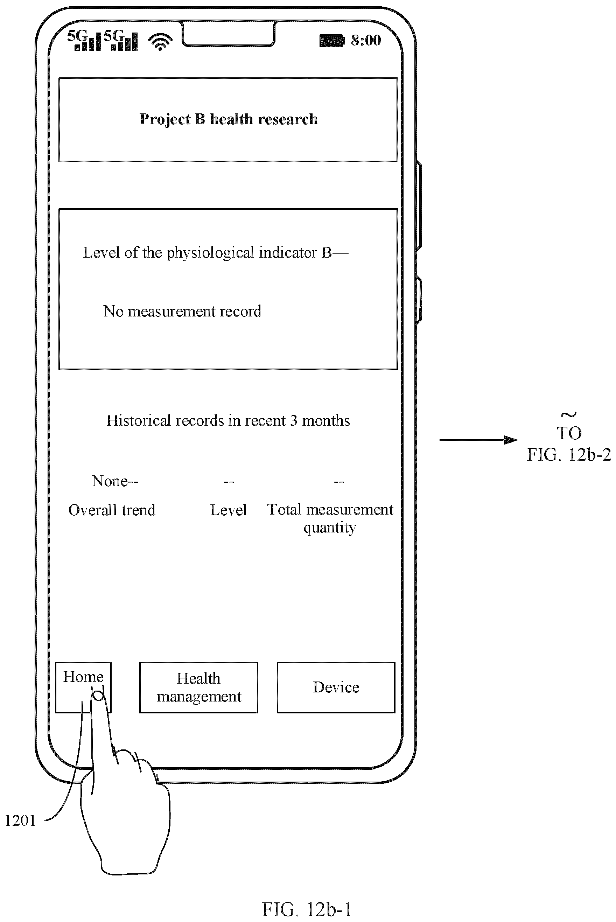

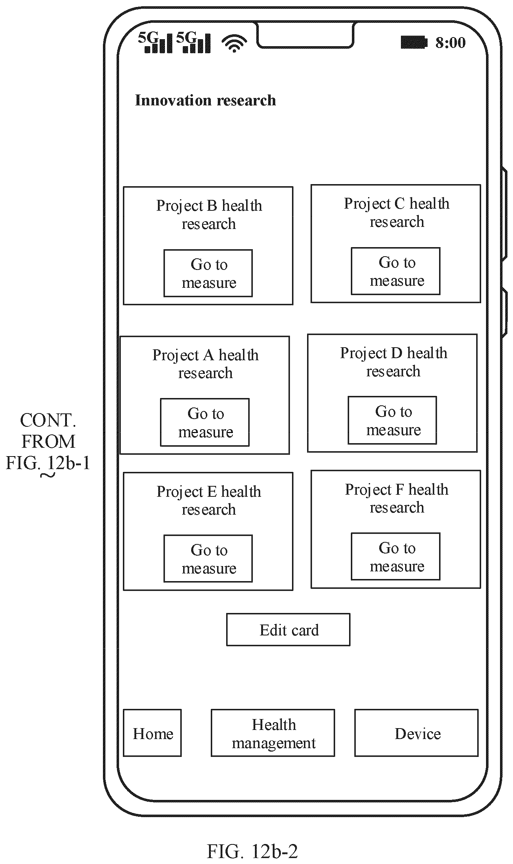

FIG. 12b-1 and FIG. 12b-2 are a diagram of another health management scenario according to an embodiment of this application;

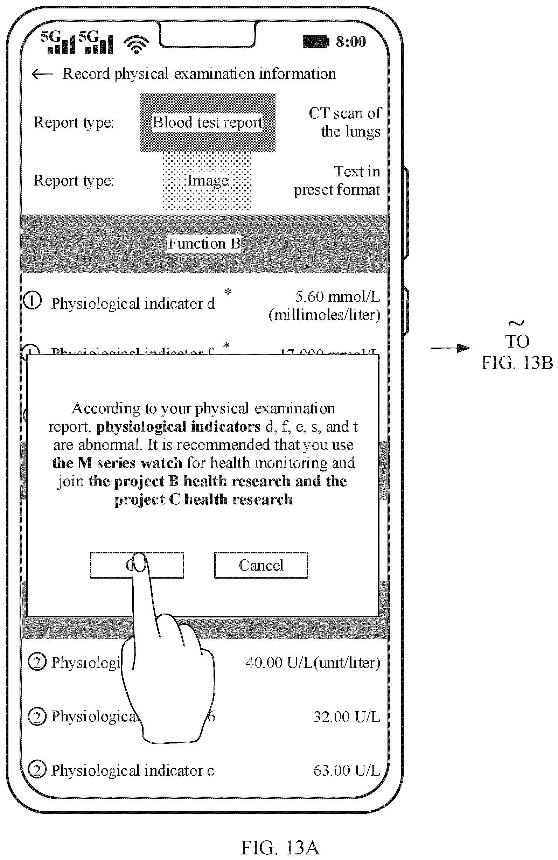

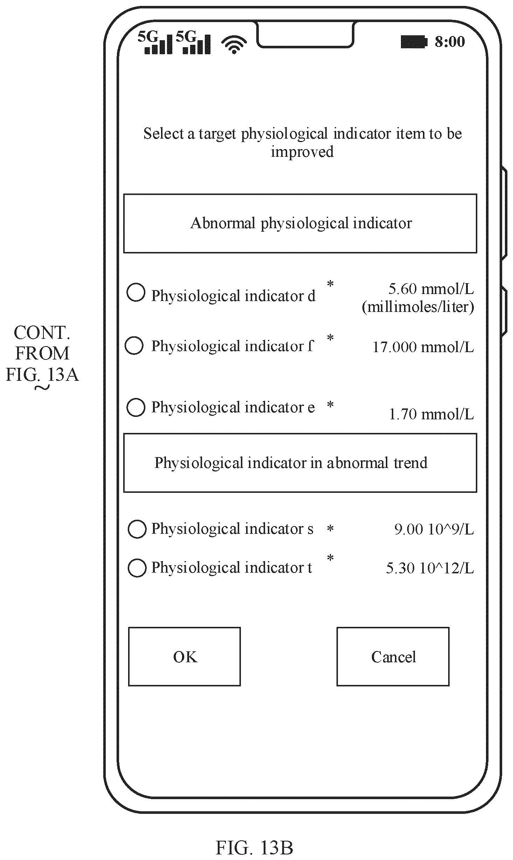

FIG. 13A and FIG. 13B are a diagram of another health management scenario according to an embodiment of this application;

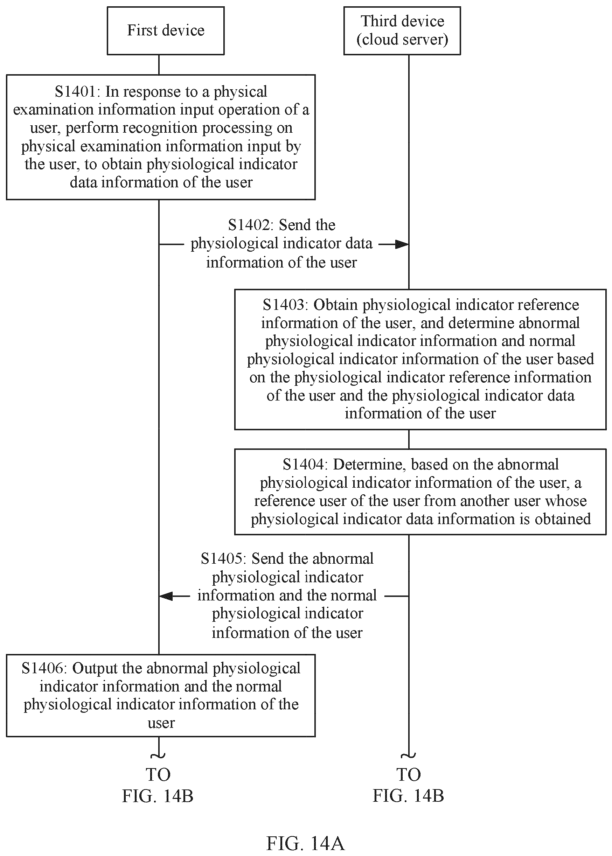

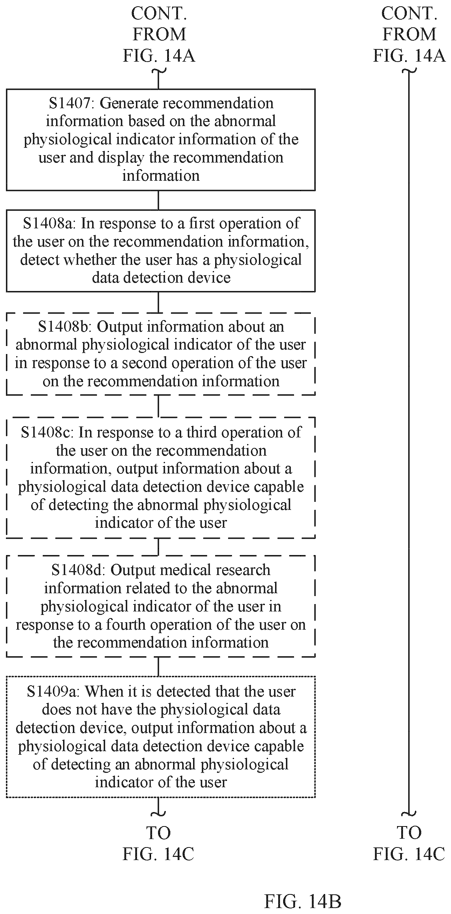

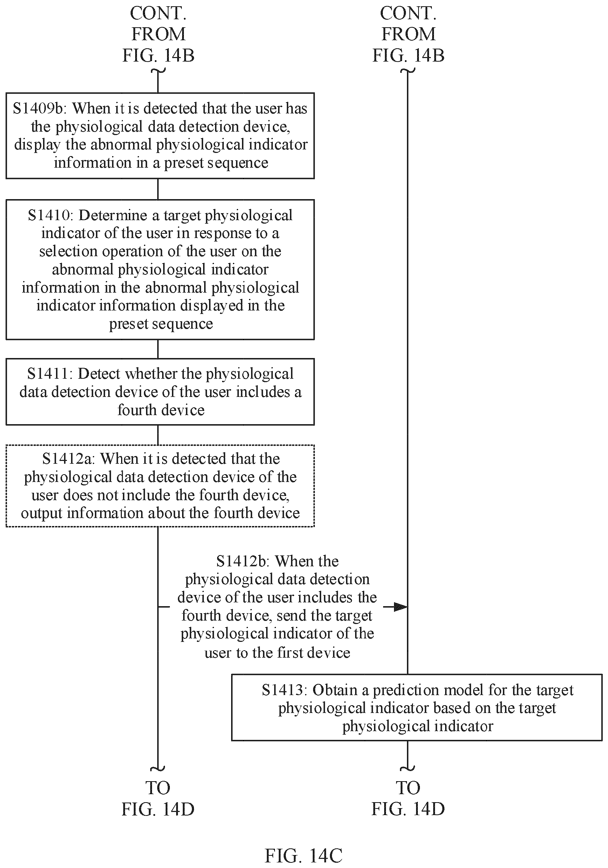

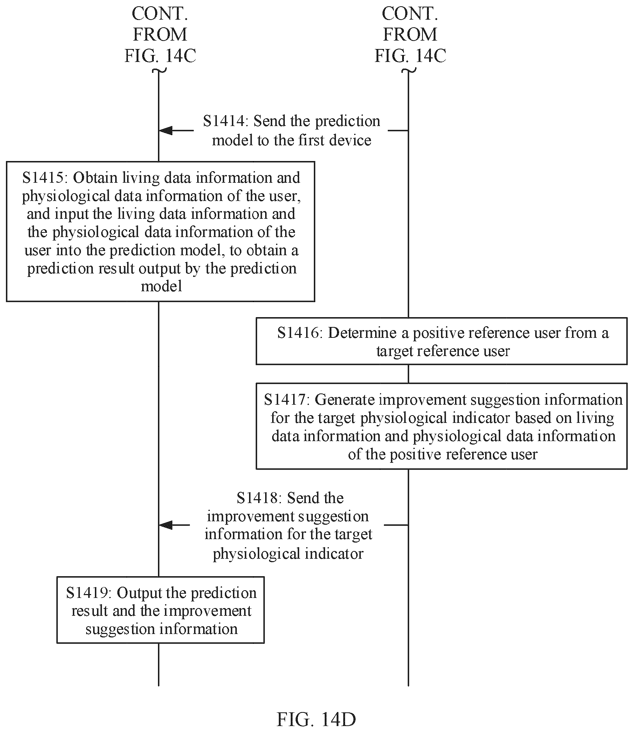

FIG. 14A to FIG. 14D are a schematic flowchart of another health management method according to an embodiment of this application;

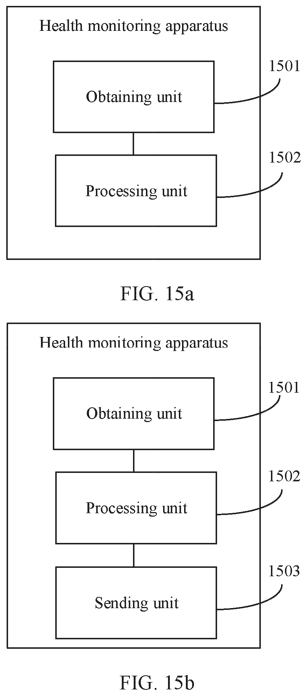

FIG. 15a is a diagram of a structure of a health management apparatus according to an embodiment of this application;
FIG. 15b is a diagram of a structure of another health management apparatus according to an embodiment of this application;

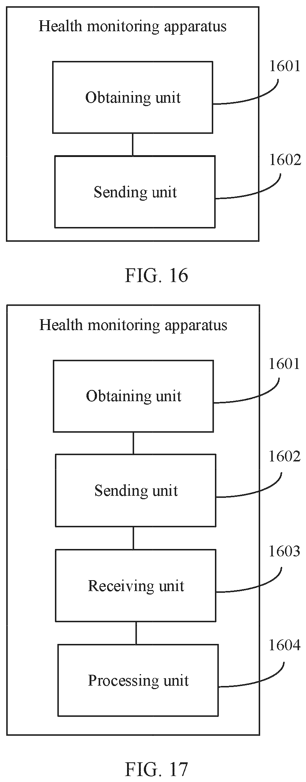

FIG. 16 is a diagram of a structure of a health management apparatus according to an embodiment of this application;
FIG. 17 is a diagram of a structure of a health management apparatus according to an embodiment of this application; and

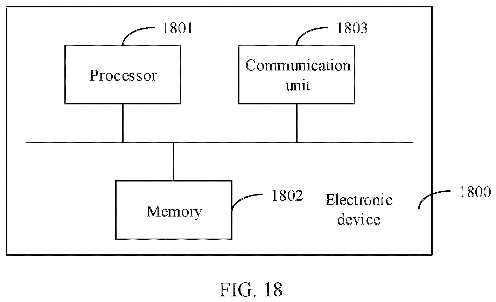

FIG. 18 is a diagram of a structure of an electronic device according to an embodiment of this application.

### DESCRIPTION OF EMBODIMENTS

To better understand technical solutions of this application, the following describes embodiments of this application in detail with reference to the accompanying drawings.

It should be noted that described embodiments are merely some rather than all of embodiments of this application. All other embodiments obtained by a person of ordinary skill in the art based on embodiments of this application without creative efforts shall fall within the protection scope of this application.

The terms used in embodiments of this application are merely for the purpose of describing specific embodiments, and are not intended to limit this application. The terms "a", "said" and "the" of singular forms used in embodiments and the appended claims of this application are also intended to include plural forms, unless otherwise specified in the context clearly.

It should be understood that the term "and/or" used in this specification describes only an association relationship between associated objects and represents that three relationships may exist. For example, A and/or B may represent the following three cases: Only A exists, both A and B exist, and only B exists. In addition, the character "/" in this specification generally indicates an "or" relationship between the associated objects.

Embodiments of this application may be applied to the field of terminal technologies. A device in the embodiments of this application may be an electronic device like a mobile phone, a tablet computer, a wearable device (for example, a watch, a band, a helmet, or a headset), a vehicle-mounted device, an augmented reality (augmented reality, AR)/virtual reality (virtual reality, VR) device, a notebook computer, an ultra-mobile personal computer (ultra-mobile personal computer, UMPC), a netbook, a personal digital assistant (personal digital assistant, PDA), or a smart home device (for example, a smart desk lamp, a smart sound box, or a smart gateway). It may be understood that a specific type of the electronic device is not limited in embodiments of this application.

Regular health examinations have become an important part of people's daily life. An existing physical examination report generally records physical examination data corresponding to a physical examination item and a normal range of the physical examination data, for example, a blood pressure value and a normal blood pressure range. A person undergoing physical examination learns a physical examination result of the person by viewing a physical examination report. If there is an abnormal indicator item in the physical examination report, the user usually adjusts a living habit, a diet, and the like, to improve the abnormal indicator item. However, whether an adjusted living habit, an adjusted diet, and the like can improve the abnormal indicator can be learned only after the user goes to a hospital again for check. Therefore, a method for evaluating whether an abnormal indicator can be improved based on living information such as a living habit and a diet of a user is urgently needed.

For the foregoing problem, embodiments of this application provide a health management method and apparatus, and a system, an electronic device, and a storage medium. A target physiological indicator of a user is determined, and a prediction model for the target physiological indicator is obtained based on the target physiological indicator. Living data information and physiological data information of the user are obtained, and the living data information and the physiological data information of the user are input into the prediction model, to obtain a prediction result output by the prediction model. The prediction result is a prediction of an impact of the living data information and the physiological data information of the user on a change trend of the target physiological indicator. In this way, in embodiments of this application, after the living data information and the physiological data information of the user are obtained, the living data information and the physiological data information may be input into the prediction model for the target physiological indicator. The prediction model may perform prediction based on the living data information and the physiological data information of the user, to obtain the prediction result. The user may learn, based on the prediction result, whether the living data information and the physiological data information of the user can improve the target physiological indicator, thereby providing a possibility of long-term automatic and intelligent physical health detection for the user, and guiding the user to healthy exercise and diet, to get rid of a potential deterioration trend, and guide optimization of the target physiological indicator. In addition, the user may perceive a change progress of a physiological indicator caused by a change of a daily living habit, learn a current status of the physiological indicator, and increase use frequency and activeness of the user. The following describes in detail.

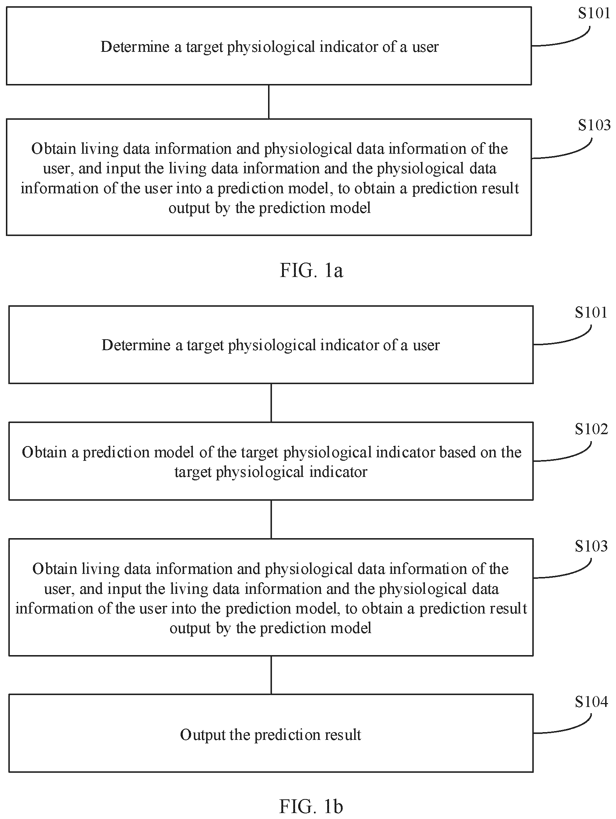

FIG. 1a and FIG. 1b are schematic flowcharts of a health management method according to an embodiment of this application. The method is applied to a first device. As shown in FIG. 1a and FIG. 1b, the method includes the following steps.

Step S101: Determine a target physiological indicator of a user.

In this embodiment of this application, when the user needs to detect an impact of a daily living habit, for example, a diet habit or a sports habit, on a change of a physiological indicator, the user may determine, as the target physiological indicator, the physiological indicator that needs to be detected for the user. In this case, the first device may obtain the target physiological indicator determined by the user.

In some embodiments, the target physiological indicator includes an indicator in a user physical examination report, for example, blood pressure and blood glucose.

It should be noted that the first device may be an electronic device that can directly interact with the user, for example, a mobile phone, or the first device may be a device that cannot be directly operated by the user, for example, a cloud server, and in this case, the user may interact with the first device by using a second device. A manner of determining the target physiological indicator of the user varies according to a different first device.

In a possible implementation, when the first device is a cloud server, the determining a target physiological indicator of a user includes: receiving the target physiological indicator of the user sent by the second device.

In other words, the target physiological indicator is determined based on selection of the user. When the first device is a cloud server, the user cannot directly send, to the cloud server, the target physiological indicator that needs to be detected for the user, or cannot directly perform an operation on the cloud server. Generally, the user may send the target physiological indicator selected by the user to the second device that interacts with the user. For example, the user may select the target physiological indicator from a physiological indicator displayed by the second device, and the second device may determine the target physiological indicator in response to the selection operation of the user. A communication connection is established between the second device and the first device, and the second device sends the target physiological indicator to the first device. The first device receives the target physiological indicator sent by the second device, and the first device obtains the target physiological indicator of the user.

In a possible implementation, when the first device is an electronic device that can be directly operated by the user, the determining a target physiological indicator of a user includes: determining the target physiological indicator of the user in response to a selection operation of the user.

In other words, the user can directly perform an operation on the first device. In this case, the user may input, to the first device, the target physiological indicator that needs to be detected for the user, or the user may input the target physiological indicator into the first device on a display interface of the first device in a selection manner when a physiological indicator of the user is pre-displayed by the first device. In this case, the first device may detect a selection operation of the user, and in response to the selection operation of the user, the first device may determine the target physiological indicator that needs to be detected for the user, to obtain the target physiological indicator of the user.

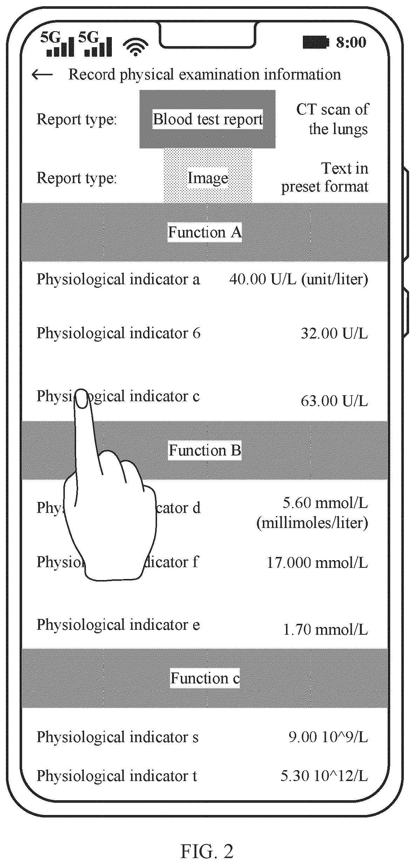

For example, it is assumed that the first device is a mobile phone. After the user inputs the physical examination report of the user into the mobile phone, after detecting data information of each physiological indicator of the user, the mobile phone may display abnormal physiological indicator data information and normal physiological indicator data information of the user, as shown in FIG. 2. The user selects a physiological indicator c as the target physiological indicator.

It should be noted that, in this embodiment of this application, the first device needs to predict, based on a prediction mode, an impact of related information about a daily living habit of the user on a change of the target physiological indicator. In some embodiments, the first device may first obtain a prediction model for the target physiological indicator based on the target physiological indicator. In this case, step S102 may be performed, as shown in FIG. 1b. In some embodiments, if the first device has obtained the prediction model, step S103 may be directly performed without performing of step S102, as shown in FIG. 1a.

Step S102: Obtain the prediction model for the target physiological indicator based on the target physiological indicator.

In this embodiment of this application, the prediction model for the target physiological indicator is a model that is pre-trained and that is used to predict the impact of the related information of the daily living habit of the user on the change of the target physiological indicator. After obtaining the target physiological indicator, the first device may obtain the prediction model for the target physiological indicator based on the target physiological indicator, to predict the target physiological indicator. In this case, a manner of obtaining the prediction model for the target physiological indicator varies according to a different first device.

In a possible implementation, when the first device is a cloud server, the obtaining the prediction model for the target physiological indicator based on the target physiological indicator includes:
determining, based on the target physiological indicator, a target reference user from a user whose physiological indicator data information, living data information, and physiological data information are obtained; and constructing the prediction model based on obtained living data information and obtained physiological data information of the target reference user.

Obtained physiological indicator data information of the target reference user includes data information of the target physiological indicator. The living data information is basic data information of the user in daily life, including daily work and rest data information, diet information, and exercise information of the user. The physiological data information is data obtained by the user by detecting a physiological indicator of the user by using a fourth device that the user wears daily.

It should be noted that the fourth device is a physiological data detection device capable of detecting the target physiological indicator. The fourth device may be an intelligent wearable device, for example, may be a device capable of detecting the physiological indicator of the user, for example, a smartwatch, a band, a body fat scale, or the like.

In this embodiment of this application, when the first device is the cloud server, to better detect health of the user, the user may upload data information of physiological indicators of the user during each physical examination, daily living data information of the user, and physiological data information of the user to the cloud server for storage. The cloud server, that is, the first device, may obtain physiological indicator data information of a plurality of users, daily living data information of the plurality of users, and physiological data information of the plurality of users. In this case, the cloud server, that is, the first device, may determine, as the target reference user based on the target physiological indicator, a user whose obtained physiological indicator data information includes the data information of the target physiological indicator from a user whose physiological indicator data information, living data information, and physiological data information are obtained. After determining the target reference user corresponding to the target physiological indicator, the cloud server, that is, the first device may train a preset network model based on the living data information, the physiological data information, and the physiological indicator data information of the target reference user. The cloud server, that is, the first device may use a trained network model as the prediction model, to obtain the prediction model.

The preset network model is a preset network model. The network model may be a Transformer model, or may be an Xgboost (extreme Gradient Boosting, extreme gradient boosting) model, or may be another network model. This is not limited in this application. 2

The target reference user includes a positive reference user and a negative reference user. The positive reference user is a user whose target physiological indicator is improved in the target reference user. The negative reference user is a user whose target physiological indicator is deteriorated in the target reference user. During training of the preset network model, living data information, physiological data information, and physiological indicator data information of both the positive reference user and the negative reference user are included. Therefore, the trained prediction model may determine an impact on the target physiological indicator based on the current living data information and the physiological data information of the user.

In a possible implementation, when the first device is an electronic device that can directly interact with the user, the obtaining the prediction model for the target physiological indicator based on the target physiological indicator includes: sending the target physiological indicator to a third device; and receiving the prediction model that is for the target physiological indicator and that is sent by the third device.

To be specific, when the first device is the electronic device that can directly interact with the user, to protect user privacy, the first device cannot directly obtain physiological indicator data information of another user, daily living data information of the another user, and physiological data information of the another user. In other words, the first device cannot directly train the prediction model. In this case, the first device may construct the prediction model by using the third device, for example, a cloud server, that can obtain the physiological indicator data information of the another user, the daily living data information of the another user, and the physiological data information of the another user. After constructing the prediction model, the third device may directly send the model to the first device, and the first device may directly use the trained prediction model. Based on this, to obtain the prediction model for the target indicator, the first device sends the target indicator to the third device. After receiving the target indicator, the third device may determine, based on the target indicator, a target reference user from a user whose physiological indicator data information, living data information, and physiological data information are obtained. Obtained physiological indicator data information of the target reference user includes data information of the target physiological indicator. The preset network model is trained based on the obtained physiological indicator data information, obtained living data information, and obtained physiological data information of the target reference user, to construct the prediction model. For details, refer to the foregoing process of obtaining the prediction model when the first device is a cloud server. Details are not described herein again. After obtaining the prediction model, the third device may send the prediction model to the first device, and the first device receives the prediction model sent by the third device, to obtain the prediction model.

It should be understood that the third device is a device that can obtain the physiological indicator data information of the another user, the daily living data information of the another user, and the physiological data information of the another user. For example, the third device is a cloud server.

Step S103: Obtain living data information and physiological data information of the user, and input the living data information and the physiological data information of the user into the prediction model, to obtain a prediction result output by the prediction model.

The prediction result represents a predicted impact of the living data information and the physiological data information of the user on the change of the target physiological indicator, and the physiological data information includes related data of the target physiological indicator.

In this embodiment of this application, the related information about the daily living habit of the user needs to be obtained for a prediction of an impact of the daily living habit of the user on the change of the target physiological indicator. In this case, that the first device needs to obtain the living data information of the user is to obtain daily diet information, daily exercise information, daily work and rest information, and the like of the user. To more accurately predict a change of the target physiological indicator, the physiological data information of the user further needs to be obtained, that is, physiological data information obtained through detection by using the fourth device. The physiological data information includes related data of the target physiological indicator. After the living data information and the physiological data information of the user are obtained, the living data information and the physiological data information of the user may be used as an input of the prediction model and input into the prediction model. The prediction model predicts a change trend of the target physiological indicator based on the input living data information and the input physiological data information of the user, to obtain the prediction result.

When the first device obtains the living data information and the physiological data information of the user, an obtaining manner varies according to a different first device.

In a possible implementation, when the first device is the cloud server, the obtaining living data information and physiological data information of the user includes: receiving the living data information and the physiological data information of the user sent by the second device.

Because the living data information of the user needs to be obtained from the user, when the first device is the cloud server, the living data information of the user cannot be directly obtained. In this case, the user may send the living data information of the user to the second device, and the second device sends the living data information of the user to the cloud server. In addition, to facilitate the user to view data detected by the fourth device at any time, the physiological data detection device is usually connected to the second device that interacts with the user in a communication connection. In this case, the second device may obtain the physiological data information of the user from the fourth device, and transmit the physiological data information to the first device, that is, the cloud server. The first device is the cloud server, and receives the living data information and the physiological data information of the user sent by the second device.

In a possible implementation, when the first device is the electronic device that can directly interact with the user, the obtaining living data information of the user includes: obtaining the living data information of the user in response to an input operation of the living data information of the user. The obtaining physiological data information of the user includes: obtaining, from the fourth device, the physiological data information of the user detected by the fourth device.

In other words, when the first device is the electronic device that can directly interact with the user, the user may directly feed back the living data information of the user by the first device. In this case, the user may input daily living data information of the user into the first device. In this case, the first device obtains the living data information of the user in response to the input operation of the living data information of the user. Because the physiological data information is detected by the fourth device after the user wears the fourth device, the first device may obtain, from the fourth device, the physiological data information of the user detected by the fourth device. In this case, the first device may actively send an obtaining request to the fourth device, and the fourth device sends the physiological data information of the user detected by the fourth device to the first device. Alternatively, the fourth device may periodically and actively send the physiological data information of the user detected by the fourth device to the first device. Certainly, another obtaining manner may be used. This is not limited in this application.

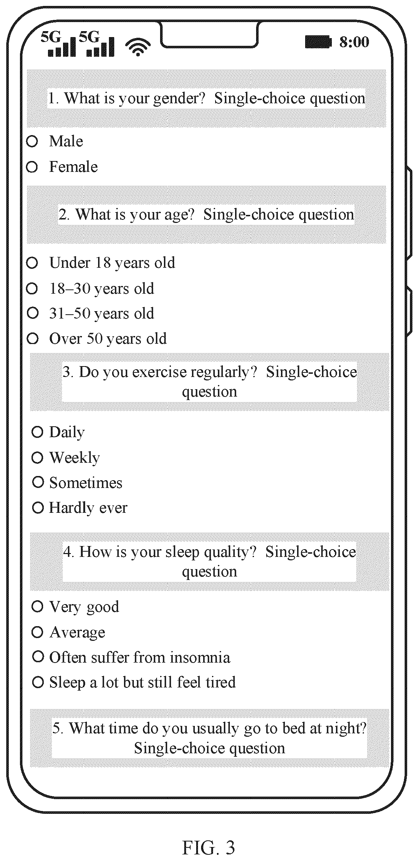

In a possible implementation, to obtain the living data information of the user more comprehensively, the first device may display, in a display device of the first device, prompt information about which living data information that the user needs to fill in. As shown in FIG. 3, the user may fill in corresponding data information according to the prompt information of the first device, to obtain more comprehensive and accurate living data information of the user.

To predict the target physiological indicator more accurately, in this case, the physiological data information obtained by the first device may be physiological data information detected after the user wears the physiological data detection device for a time longer than a first preset time threshold. To be specific, after the user wears the fourth device for a time longer than the first preset time threshold, the first device obtains the physiological data information detected by the fourth device.

It should be understood that the first preset time threshold is preset according to an actual requirement. For example, the first preset time threshold is 24 hours. In this case, the user needs to wear the physiological data detection device over 24 hours, and the first device obtains physiological data information detected by the physiological data detection device on the user for at least 24 hours.

Further, to have a more accurate prediction on the target physiological indicator, the physiological data information may be obtained by the first device at a time that is between a time when the fourth device detects the physiological data information and a time when the user needs to predict the target physiological indicator and that does not exceed a second preset time threshold. In other words, the first device needs to obtain recent physiological data information of the user. In this case, the second preset time threshold may be set, and all physiological data information is detected within the second preset time threshold prior to a time when the user needs to predict the target physiological indicator.

It should be understood that the second preset time threshold is preset according to an actual requirement.

In some embodiments, after obtaining the prediction result, the first device may output the prediction result obtained by the first device, so that the user learns the prediction result. In this case, the following step S104 may be performed, as shown in FIG. 1b.

Step S104: Output the prediction result.

In this embodiment of this application, after obtaining the prediction result, the first device may output the prediction result, so that the user learns the prediction result through the prediction result output by the first device. A manner of outputting the prediction result varies according to a different first device.

In a possible implementation, when the first device is the cloud server, the outputting the prediction result includes: sending the prediction result to the second device, so that the second device outputs the prediction result to the user.

To be specific, when the first device is the cloud server, if the cloud server outputs a preset result, the user cannot directly obtain the preset result. Therefore, the first device may send the preset result to the second device, and the second device outputs the prediction result to the user. For example, the second device may display the prediction result through a display device of the second device, and the user learns the prediction result through content displayed on the display device. Alternatively, the second device may output the prediction result to the user in a voice broadcast manner. Certainly, the second device may alternatively output the prediction result in another manner. This is not limited in this application.

In a possible implementation, when the first device is the electronic device that can directly interact with the user, the first device may output the prediction result in a display manner, a voice broadcast manner, or another manner.

In this way, in embodiments of this application, after the living data information and the physiological data information of the user are obtained, the living data information and the physiological data information may be input into the prediction model for the target physiological indicator. The prediction model may perform prediction based on the living data information and the physiological data information of the user, to obtain the prediction result. The user may learn, based on the prediction result, whether the living data information and the physiological data information of the user can improve the target physiological indicator, thereby providing a possibility of long-term automatic and intelligent physical health detection for the user, and guiding the user to healthy exercise and diet, to get rid of a potential deterioration trend, and guide optimization of the target physiological indicator. In addition, the user may perceive a change progress of a physiological indicator caused by a change of a daily living habit, learn a current status of the physiological indicator, and increase use frequency and activeness of the user.

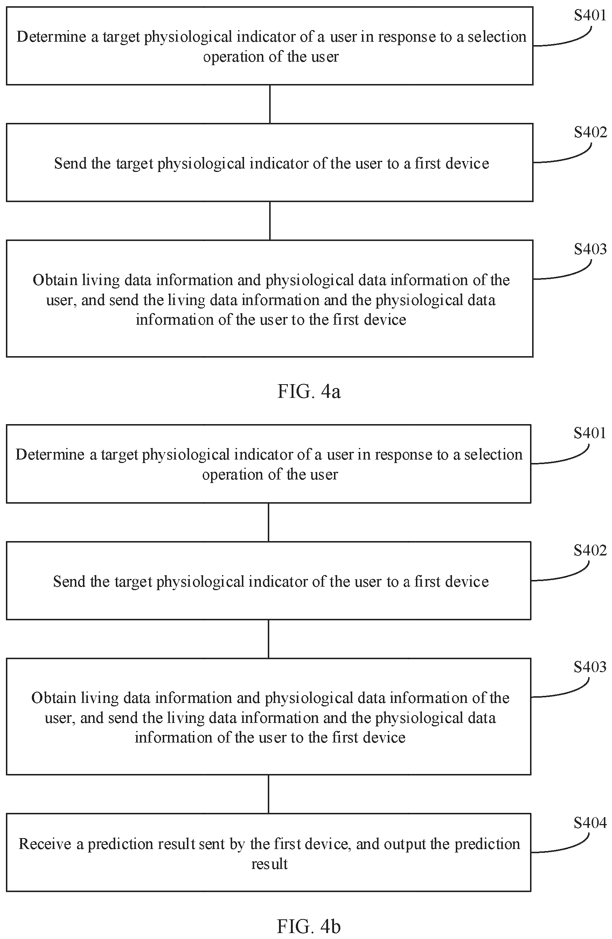

FIG. 4a and FIG. 4b are schematic flowcharts of a health management method according to an embodiment of this application. The method is applied to a second device. As shown in FIG. 4a and FIG. 4b, the method includes the following steps.

Step S401: Determine a target physiological indicator of a user in response to a selection operation of the user.

In this embodiment of this application, when the user needs to detect an impact of a daily living habit, for example, a diet habit or a sports habit, on a change of a physiological indicator, the user may determine, as the target physiological indicator, the physiological indicator that needs to be detected for the user. The second device is an electronic device that can interact with the user. In this case, the user may directly set the target physiological indicator in the second device. For example, the user may input the target physiological indicator to the second device on a display interface of the second device in a selection manner when a physiological indicator of the user is pre-displayed by the second device. In this case, the second device may detect a selection operation of the user, and in response to the selection operation of the user, the second device may determine the target physiological indicator that needs to be detected for the user, to obtain the target physiological indicator of the user.

For details, refer to step S101. Details are not described herein again.

Step S402: Send the target physiological indicator of the user to a first device.

In this embodiment of this application, after obtaining the target physiological indicator, the second device may send the target physiological indicator to the first device, and the first device predicts an impact of a living habit of the user on the target physiological indicator. Therefore, the second device needs to send the target physiological indicator to the first device, to notify the first device of a specific physiological indicator that needs to be detected.

After receiving the target physiological indicator, the first device may construct a prediction model for the target physiological indicator based on the target physiological indicator. The prediction model for the target physiological indicator is a model that is pre-trained and that is used to predict an impact of related information of a daily living habit of the user on a change of the target physiological indicator. For details, refer to step S102. Details are not described herein again.

Step S403: Obtain living data information and physiological data information of the user, and send the living data information and the physiological data information of the user to the first device.

For details, refer to step S103. Details are not described herein again.

In a possible implementation, the obtaining living data information of a user includes: obtaining the living data information of the user in response to an input operation of the living data information of the user. For details, refer to step S103. Details are not described herein again.

In a possible implementation, the obtaining physiological data information of a user includes: obtaining, from a fourth device, the physiological data information of the user monitored by the fourth device. For details, refer to step S103. Details are not described herein again.

In some embodiments, the first device may send the prediction result obtained by the first device to the second device, and the second device outputs the prediction result. In this case, the following step S404 may be performed, as shown in FIG. 4b.

Step S404: Receive the prediction result sent by the first device, and output the prediction result.

In this embodiment of this application, after the second device sends the target physiological indicator to the first device, the first device constructs the prediction model for the target physiological indicator based on the target physiological indicator. After obtaining the living data information and the physiological data information of the user, the second device sends the living data information and the physiological data information of the user to the first device, and the first device predicts, based on the living data information and the physiological data information of the user, the impact of the living habit of the user on the change of the target physiological indicator by using a prediction model for the target physiological indicator, to obtain the prediction result. After obtaining the prediction result, the first device sends the prediction result to the second device. After receiving the prediction result, the second device outputs the prediction result, to notify the user of the impact of the living habit of the user on the change of the target physiological indicator.

In a possible implementation, when outputting the prediction result, the second device may directly display the prediction result on a display device, that is, display the prediction result through the display device. Alternatively, the second device may broadcast the prediction result in a voice broadcast manner. Certainly, the second device may alternatively output the prediction result in another manner. This is not limited in this application.

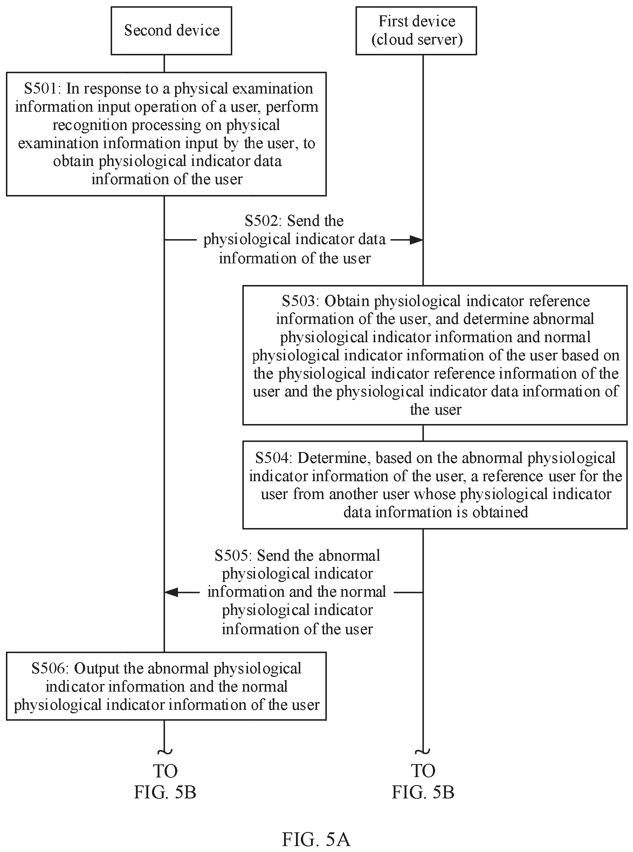

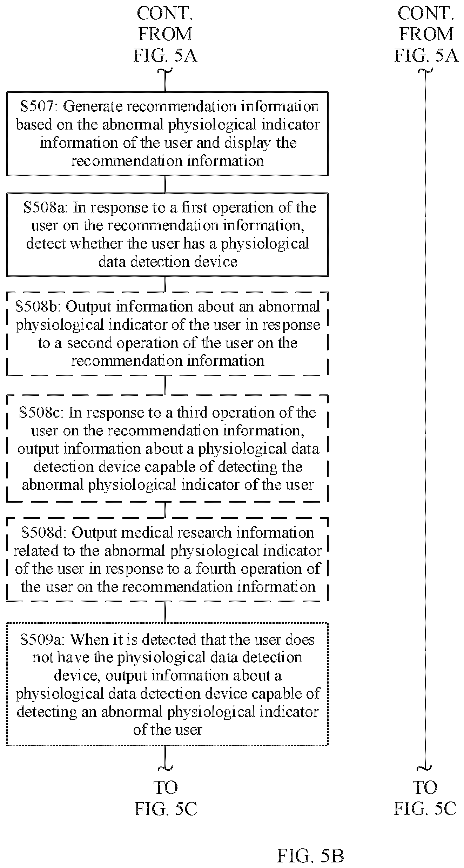

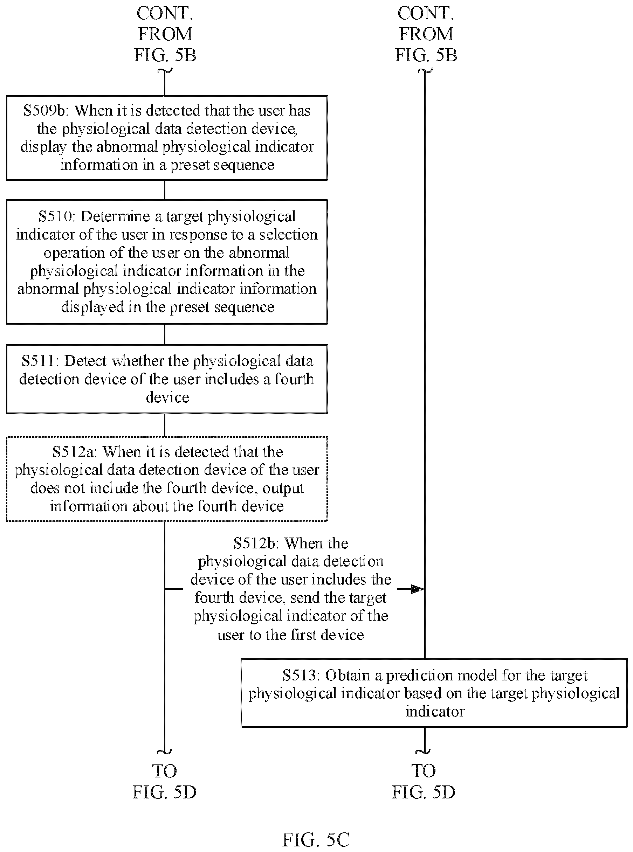

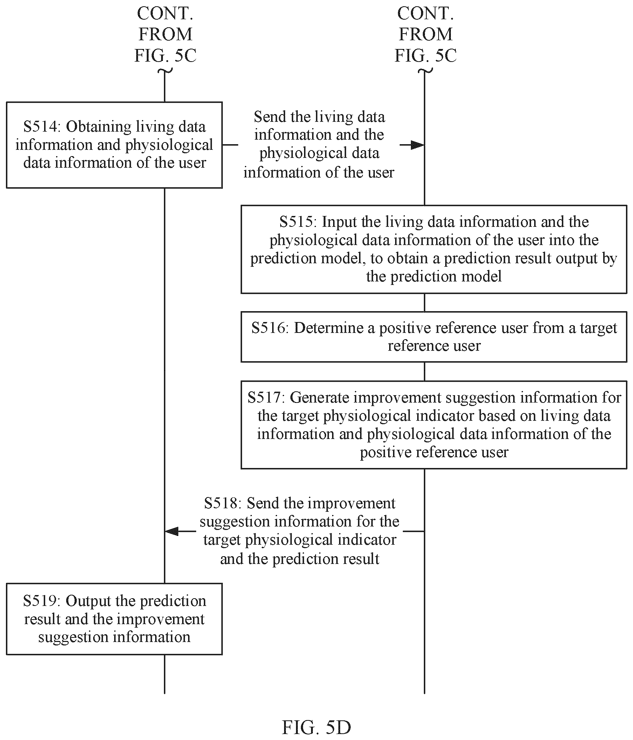

FIG. 5A to FIG. 5D are a schematic flowchart of a health management method according to an embodiment of this application. The first device may be a cloud server, or may be an electronic device that can directly interact with a user. In this embodiment of this application, an example in which the first device is a cloud server is used for description. As shown in FIG. 5A to FIG. 5D, the method includes the following steps.

Step S501: In response to a physical examination information input operation of a user, a second device performs recognition processing on physical examination information input by the user, to obtain physiological indicator data information of the user.

In this embodiment of this application, to more accurately predict a change of the physiological indicator of the user, a physical examination report of the user needs to be first obtained. In this case, the user may record the physical examination report of the user into the second device. For example, the user may upload the physical examination report of the user to the second device in an image form, or upload the physical examination report of the user to the second device in texts in a preset format. Alternatively, the user may input the physical examination report of the user into the second device in a text form. The second device receives experience information of the user in response to a physical examination information input operation of the user. The second device performs recognition processing on the physical examination information input by the user, to obtain physiological indicator data information of the user in the physical examination information.

In a possible implementation, the second device may perform, by using a template extraction algorithm or an OCR (Optical Character Recognition, optical character recognition) recognition algorithm, text recognition on the physical examination information input by the user, to obtain the physiological indicator data information of the user. For example, the user uploads the physical examination report of the user to the second device in an image form. After receiving the physical examination information in the image format, the second device may use an OCR recognition algorithm to perform text recognition on the physical examination information in the image format, to obtain the physiological indicator data information of the user.

After receiving the physical examination information of the image format, the second device may pre-process the physical examination information of the image format, for example, perform processing such as binarization, noise removal, and tilt correction, to obtain a document picture, perform layout analysis, character cutting, and character recognition processing on the document picture, to recognize text information, and perform processing such as layout restoration and verification on the recognized text information, to finally obtain needed text information, that is, obtain data information of each physiological indicator of the user.

Alternatively, when the physical examination report uploaded by the user is in texts in a preset format, the second device may recognize text information in a template extraction manner, to obtain data information of each physiological indicator of the user. In other words, the second device may detect a physical examination report file in a predetermined format that is input by the user, and obtain modules included in the physical examination report, to extract physiological indicator data information of the user from the modules.

In a possible implementation, the text in the preset format may be a text in a PDF format. Certainly, the text may alternatively be in another format. This is not limited in this application.

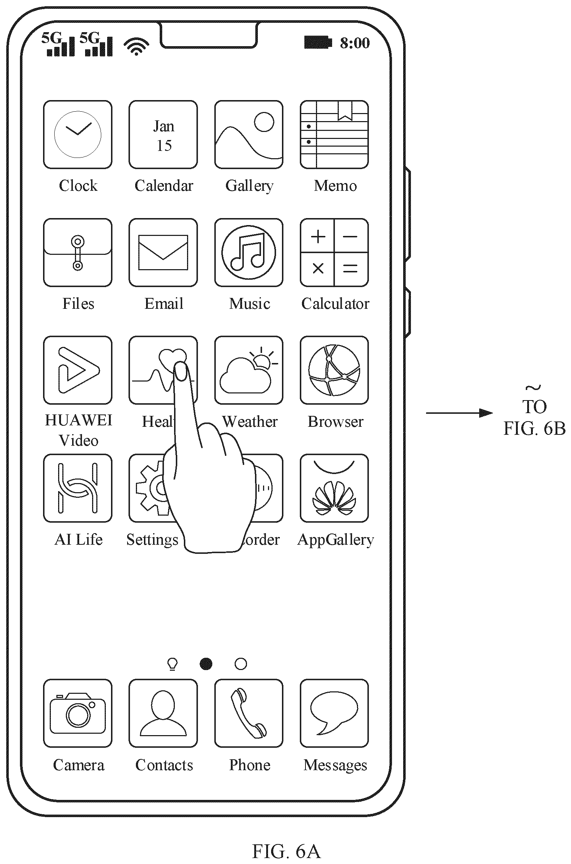

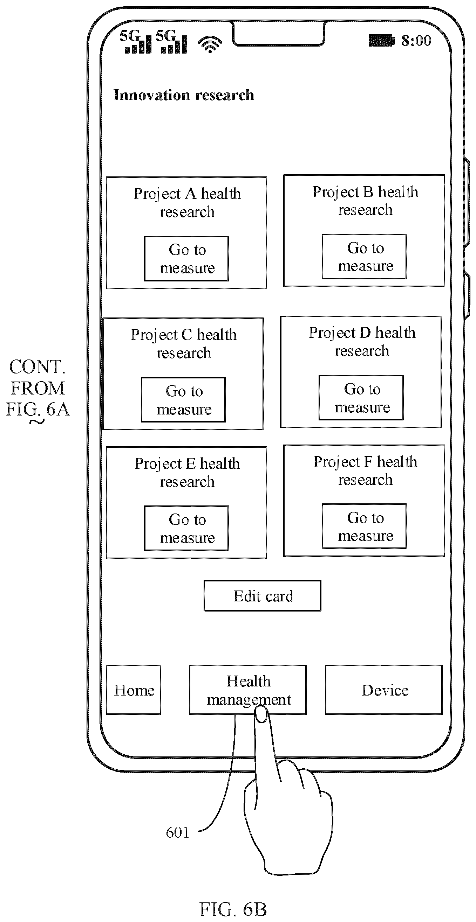

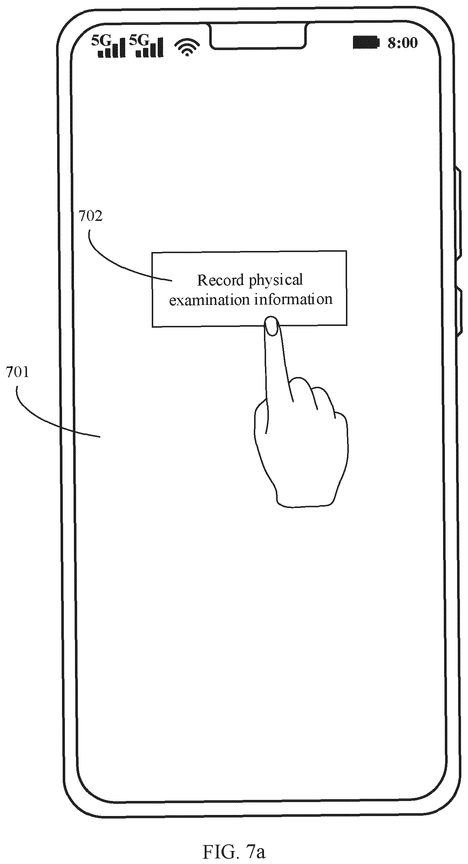

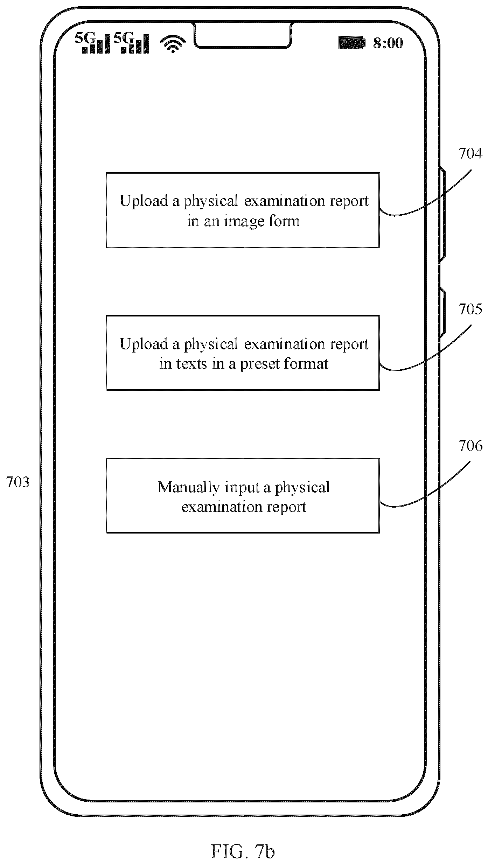

For example, an example in which the second device is a mobile phone and the first device is a cloud server is used for description. When the user needs to perform health management, the user may tap a health management application to enter a home page of the health management application, as shown in FIG. 6A and FIG. 6B. The home page of the health management application includes a health management control 601. If the user needs to perform health management, in response to the user operating the health management control 601, the mobile phone displays an interface 701 shown in FIG. 7a. The interface 701 includes a physical examination information recording control 702. In response to an operation of the user on the physical examination information recording control 702, the mobile phone displays an interface 703 shown in FIG. 7b. The interface 703 includes a control 704 for uploading a physical examination report in an image form, a control 705 for uploading a physical examination report in texts in a preset format, and a control 706 for manually inputting a physical examination report. It is assumed that the user uploads a physical examination report in an image form. In response to the user operating the control 704 for uploading a physical examination report in an image form, the mobile phone obtains the physical examination report in the image form. After obtaining the physical examination report in the image form, the mobile phone may perform OCR recognition on the physical examination report in the image form, to obtain physiological indicator data information of the user.

It should be noted that the physical examination report may be uploaded in another manner. This is not limited in this application. The project A health research, the project B health research, the project C health research, the project D health research, the project E health research, and the project F health research in FIG. 6A and FIG. 6B may be a respiratory health research, a blood pressure health research, a heart health research, a liver fat health research, and the like, or certainly may be another health research. The health research is set according to an actual requirement. In addition, a quantity of health research projects that can be performed in the second device may be more or fewer. Content, a quantity, and the like of health researches are not limited in this application.

It should be understood that, in embodiments of this application, content shown in FIG. 6A and FIG. 6B to FIG. 13A and FIG. 13B is an example, and does not limit the content of the embodiments of this application. In actual application, the content shown in FIG. 6A and FIG. 6B to FIG. 13A and FIG. 13B may be adjusted according to an actual requirement. This is not limited in this application.

Step S502: The second device sends the physiological indicator data information of the user to the first device. The first device receives the physiological indicator data information of the user sent by the second device.

In this embodiment of this application, to reduce workload of the second device, the second device may not perform analysis-related processing on a physiological indicator of the user, but the processing is performed by the cloud server. In this case, the second device needs to send, to the first device, that is, the cloud server, the physiological indicator data information of the user obtained by the second device.

In a possible implementation, to better protect user privacy, before sending the physiological indicator data information of the user to the first device, the second device may output a query message indicating whether to upload the physical examination information to the cloud server. When a message indicating that the user agrees to upload the physical examination information to the cloud server is obtained, the physiological indicator data information of the user is sent to the first device.

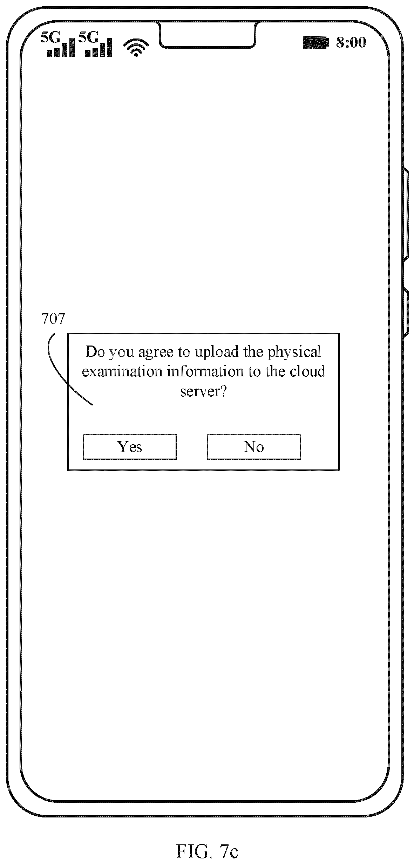

As described in the foregoing example, after obtaining the physiological indicator data information of the user, the mobile phone may display an interface shown in FIG. 7c. The interface shown in FIG. 7c includes prompt information 707, and the prompt information 707 is text information. For example, the text information is "Do you agree to upload the physical examination information to the cloud server?". In some embodiments, if the user selects a "Yes" control, the mobile phone uploads the obtained physiological indicator data information of the user to the cloud server. If the user selects a "No" control, in some embodiments, the mobile phone may return to the home page of the health management application.

Step S503: The first device obtains physiological indicator reference information of the user, and determines abnormal physiological indicator information and normal physiological indicator information of the user based on the physiological indicator reference information of the user and the physiological indicator data information of the user.

In this embodiment of this application, after receiving the physiological indicator data information of the user sent by the second device, the first device, that is, the cloud server, may obtain the physiological indicator reference information corresponding to the user. In some embodiments, the first device may use a standard reference range of each physiological indicator as the physiological indicator reference information of the user. In some embodiments, a standard reference range of each physiological indicator is set for the ordinary public. However, due to an impact of different living environments, data of some physiological indicators of a body may not be within the standard reference range, but the physiological indicators of the body are normal and have no abnormality. For example, residents living in a plateau area have many erythrocytes in their physiological indicators, and a quantity of the erythrocytes may exceed a standard reference range. Because of a thin oxygen amount in the plateau area, residents in the plateau area have many erythrocytes. If the erythrocytes exceed the standard reference range, it is determined that the indicator is normal. Therefore, to more accurately recognize abnormal physiological indicators and normal physiological indicators of different users, during obtaining of physiological indicator reference information of a user, the physiological indicator reference information of the user may be determined based on historical physical examination information of the user and a standard reference range of each physiological indicator. When the first device does not store historical physical examination information of the user, a standard reference range of each physiological indicator is directly determined as the physiological indicator reference information of the user.

The historical physical examination information of the user is physiological indicator data information of the user that is obtained by the cloud server before the cloud server currently obtains physiological indicator data information of the user.

It should be understood that a standard reference range of a physiological indicator is a general reference range of the physiological indicator, and each physiological indicator in the human body has a corresponding standard reference range. Generally, if data of a physiological indicator is within a standard reference range, it is determined that the physiological indicator is normal; or if data of a physiological indicator is not within a standard reference range, it is determined that the physiological indicator is abnormal.

When the first device stores the historical physical examination information of the user, that is, when the first device stores historical physiological indicator data information of the user, the first device may determine, based on the historical physiological indicator data information of the user and the standard reference range of each physiological indicator, a reference range of each physiological indicator corresponding to the user. In some embodiments, if the first device stores at least two pieces of historical physical examination information of the user, and data of a physiological indicator a in each piece of historical physical examination information is less than a standard reference range of the physiological indicator a and is not greater than a first preset threshold, the first device may determine a reference range of the physiological indicator a of the user is [a1, a2]. a1 is a minimum value that corresponds to the physiological indicator a corresponding to the user and that is determined by the first device, and a difference between a1 and a minimum value of the standard reference range of the physiological indicator a is not greater than the first preset threshold. a2 is a maximum value that corresponds to the physiological indicator a corresponding to the user and that is determined by the first device, and a difference between a2 and a maximum value of the standard reference range of the physiological indicator a is not greater than a second preset threshold. Certainly, if the user detects at least one physiological indicator of the user by using a physiological data detection device, and uploads detected data to the first device, the first device may determine, based on historical physiological indicator data information of the user, standard reference range of each physiological indicator, and physiological indicator data information detected by the physiological data detection device, a reference range of each physiological indicator corresponding to the user.

In conclusion, the first device may determine, based on the historical physiological indicator data information of the user and the standard reference range of each physiological indicator, the reference range of each physiological indicator corresponding to the user, to obtain the physiological indicator reference information of the user. In this way, physiological indicator reference information of each user may be determined for different users, that is, personalized physiological indicator reference information of the user is determined, and subsequent recognition of an abnormal physiological indicator or a physiological indicator with an abnormal trend of the user is more accurate.

After obtaining the physiological indicator reference information of the user, the first device may determine, based on the physiological indicator reference information of the user and the physiological indicator data information of the user, whether the data information of each physiological indicator of the user is abnormal, or whether each physiological indicator of the user has an abnormal trend. For example, if a physiological indicator b of the user is greater than or less than a reference range of the physiological indicator b in the physiological indicator reference information of the user, it may be determined that the physiological indicator b of the user is abnormal. Alternatively, a physiological indicator b of the user increases or decreases in comparison with a physiological indicator b in the historical medical examination information, and a value of the physiological indicator b is at an edge of a reference range of the physiological indicator b in the physiological indicator reference information of the user. This helps a difference between the value of the physiological indicator b and a maximum value of the reference range of the physiological indicator b in the physiological indicator reference information of the user be less than a third preset threshold, and it may be determined that the physiological indicator b has an abnormal trend.

The first device determines, based on the data information of each physiological indicator, whether each physiological indicator is abnormal or whether there is an abnormal trend, and determines the abnormal physiological identification information and the normal physiological indicator information of the user. The abnormal physiological indicator information includes information about an abnormal physiological indicator and/or information about a physiological indicator with an abnormal trend.

In a possible implementation, the abnormal physiological identification information includes value information of each abnormal physiological indicator and physiological indicator reference information of the user corresponding to each abnormal physiological indicator. The normal physiological indicator information includes value information of each normal physiological indicator and physiological indicator reference information of the user corresponding to each normal physiological indicator.

Step S504: The first device determines, based on the abnormal physiological indicator information of the user, a reference user for the user from another user whose physiological indicator data information is obtained.

In this embodiment of this application, after determining the abnormal physiological indicator information of the user, the first device may determine a reference user for the user from another user whose physiological indicator data information is obtained and whose abnormal physiological indicator information and normal physiological indicator information are determined. In other words, a reference user having a same physiological feature as the user is determined from the another user whose physiological indicator data information is the obtained. In some embodiments, the first device may determine, for each abnormal physiological indicator of the user, a reference user corresponding to each abnormal physiological indicator from the another user whose physiological indicator data information is obtained. To be specific, if the user has an abnormal physiological indicator s and an abnormal physiological indicator f, the first device determines, for the abnormal physiological indicator s of the user, a reference user corresponding to the abnormal physiological indicator s from another user whose physiological indicator data information is obtained. A physiological indicator s of another user in the reference user is abnormal or was abnormal. The first device determines, for the abnormal physiological indicator f of the user, a reference user corresponding to the abnormal physiological indicator f from the another user whose physiological indicator data information is obtained. A physiological indicator f another user in the reference user is abnormal or was abnormal.

Step S505: The first device sends the abnormal physiological indicator information and the normal physiological indicator information of the user to the second device. The second device receives the abnormal physiological indicator information and the normal physiological indicator information of the user sent by the first device.

In this embodiment of this application, after determining the abnormal physiological identification information and the normal physiological indicator information of the user, the first device may send the abnormal physiological indicator information and the normal physiological indicator information of the user to the second device, and the second device receives the abnormal physiological indicator information and the normal physiological indicator information.

Step S506: The second device outputs the abnormal physiological indicator information and the normal physiological indicator information of the user.

In this embodiment of this application, after receiving the abnormal physiological indicator information and the normal physiological indicator information of the user, the second device may output the abnormal physiological indicator information and the normal physiological indicator information, to notify the user of specific physiological indicators that are abnormal or that have an abnormal trend. In this case, the second device may output the abnormal physiological indicator information and the normal physiological indicator information in a display manner. For example, the second device displays the abnormal physiological indicator information and the normal physiological indicator information of the user on a display device. Alternatively, the second device may output the abnormal physiological indicator information and the normal physiological indicator information of the user in a voice broadcast manner. Certainly, the second device may alternatively output the abnormal physiological indicator information and the normal physiological indicator information in another manner. This is not limited in this application.

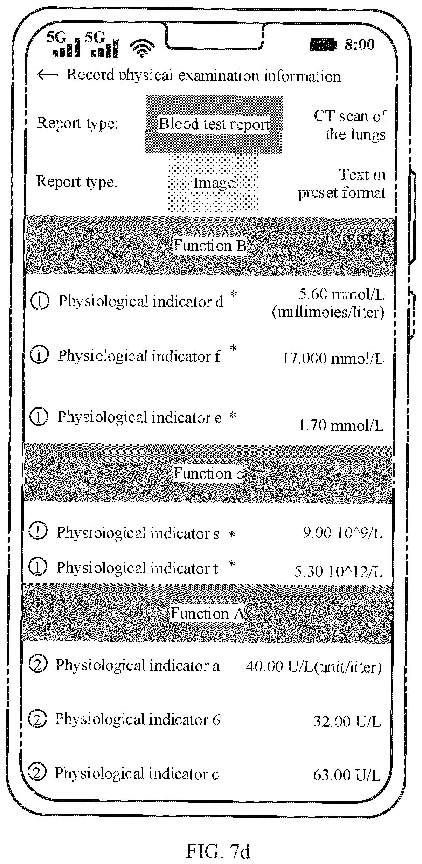

For example, as described in the foregoing example, the cloud server determines abnormal physiological indicator information and normal physiological indicator information of the user, and may send the abnormal physiological indicator information and the normal physiological indicator information of the user to the mobile phone. The mobile phone may display the received abnormal physiological indicator information and the received normal physiological indicator information through a display, such as a display interface shown in FIG. 7d, so that the user learns which physiological indicators are abnormal. The display interface shown in 7d includes the abnormal physiological indicator information and the normal physiological indicator information of the user. To help the user quickly learn which physiological indicators are abnormal physiological indicators and which physiological indicators are normal physiological indicators, abnormal physiological indicator information and normal physiological indicator information may be marked by different markers. For example, in FIG. 7d, the abnormal physiological indicator is marked by *. FIG. 7d is merely a manner of marking abnormal physiological indicator information and normal physiological indicator information. The abnormal physiological indicator information and the normal physiological indicator information may alternatively be marked in another manner. For example, the abnormal physiological indicator information is displayed in red font, and the normal physiological indicator information is displayed in green font. Alternatively, another manner is not limited in this application.

In a possible implementation, to more quickly learn a location of each physiological indicator in the physical examination report of the user, the location of each physiological indicator in the physical examination may be displayed on a circumferential side of the physiological indicator, for example, the location indicates a page in the physical examination report, as shown in FIG. 7d.

It should be understood that an arrangement and representation form of the interface shown in FIG. 7d is merely an example, and a presentation form and an annotation form of the abnormal physiological indicator information are not limited to the manner shown in FIG. 7d.

In a possible implementation, the abnormal physiological identification information includes value information of each abnormal physiological indicator and physiological indicator reference information of the user corresponding to each abnormal physiological indicator. When the normal physiological indicator information includes value information of each normal physiological indicator and physiological indicator reference information of the user corresponding to each normal physiological indicator, the user may view detailed information about each physiological indicator. In this case, in response to a details viewing operation on a physiological indicator of the user, the second device displays physiological indicator data information.

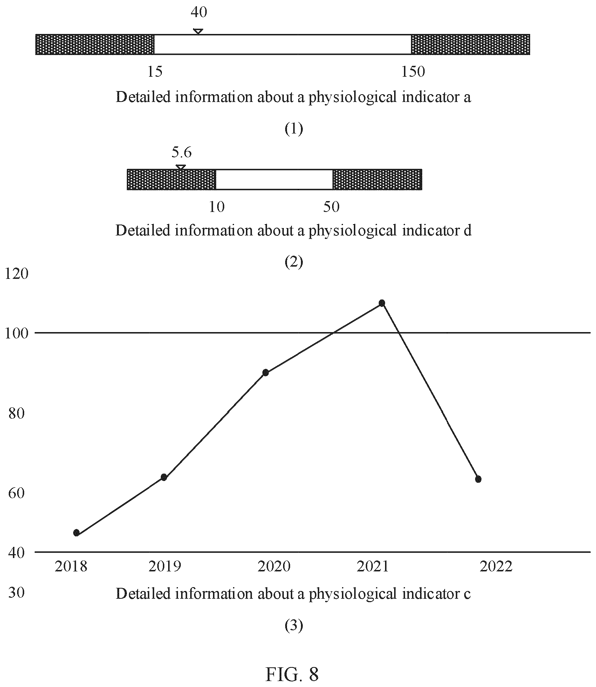

In other words, to enable the user to more accurately learn information about a physiological indicator of the user, when the user needs to view detailed information about the physiological indicator, the user may perform a details viewing operation on the physiological indicator in the second device. In this case, the second device may display value information of the physiological indicator and physiological indicator reference information corresponding to the physiological indicator. For example, when the physical examination information input by the user in step S501 is physical examination information input by the user for the first time, when the physiological indicator whose detailed information needs to be viewed by the user is a normal physiological indicator, for example, a physiological indicator a in FIG. 7d, a value of the physiological indicator in the physical examination information currently input by the user is compared with a marked reference range of the indicator. It is assumed that the marked reference range of the indicator a is 15 U/L (unit/liter) to 150 U/L, as shown in (1) in FIG. 8. It is assumed that a value of the physiological indicator a is 40.00 U/L. In this case, the value 40 of the physiological indicator a is within the standard reference range. If the physiological indicator whose detailed information needs to be viewed by the user is an abnormal physiological indicator, for example, a physiological indicator d, and it is assumed that a standard range of the physiological indicator d is 10 mmol/L (millimoles/liter) to 50 mmol/L, and a value of the physiological indicator d is 5.6 mmol/L, the value of the physiological indicator is outside the standard reference range of the indicator, as shown in (2) in FIG. 8.

Alternatively, if the physical examination information input by the user in step S501 is the physical examination information that is not input for the first time, when the user needs detailed information about a physiological indicator, a value of the physiological indicator in the physical examination information obtained in step S501, a value of the physiological indicator in the historical physical examination information, and a reference range of the physiological indicator corresponding to the user may be displayed. For example, the user needs to view detailed information about a physiological indicator c. It is assumed that the first device determines that a reference range of the physiological indicator c is 40.00 U/L to 100.00 U/L, and values of the physiological indicator c in the historical physical examination information are respectively 45, 65, 90, and 112, as shown in (3) in FIG. 8. By displaying the physiological indicator in detail, the user can more accurately learn a change of the physiological indicator of the user, thereby facilitating better management and control of the user.

In a possible implementation, the details viewing operation on the physiological indicator of the user may be a tap of the user on a specific location at the physiological indicator. For example, the user taps the physiological indicator, taps a value of the physiological indicator, or double-taps the physiological indicator. Certainly, the details viewing operation may alternatively be another operation. This is not limited in this application.

Step S507: The second device generates recommendation information based on the abnormal physiological indicator information of the user and displays the recommendation information.

The recommendation information includes at least one of information about a physiological data detection device capable of detecting an abnormal physiological indicator of the user and medical research information related to the abnormal physiological indicator of the user.

In this embodiment of this application, to provide a more comprehensive service for the user, the second device may generate the recommendation information based on the abnormal physiological information of the user, to recommend, to the user, a physiological data detection device that is more suitable for the user, and/or recommend, to the user, a medical research project that is of each hospital that is more suitable for the user, for the user to join the medical research project. In other words, after obtaining the abnormal physiological information of the user, the second device may determine the abnormal physiological indicator of the user. In this case, the second device may obtain a detection function of each physiological data detection device through a network, to determine a physiological data detection device capable of detecting an abnormal physiological indicator of the user. Similarly, the second device may obtain medical research project information of each hospital through the network, or the second device obtains medical research project information of each hospital from another device. The second device finds, from the medical research project information of each hospital, the medical research project information corresponding to the abnormal physiological indicator of the user, to generate the recommendation information. The recommendation information includes at least one of information about a physiological data detection device capable of detecting an abnormal physiological indicator of the user and medical research information related to the abnormal physiological indicator of the user.

In a possible implementation, to better learn, by the user, which abnormal physiological indicators are correspondingly recommended by the recommendation information, the recommendation information further includes the abnormal physiological indicators.

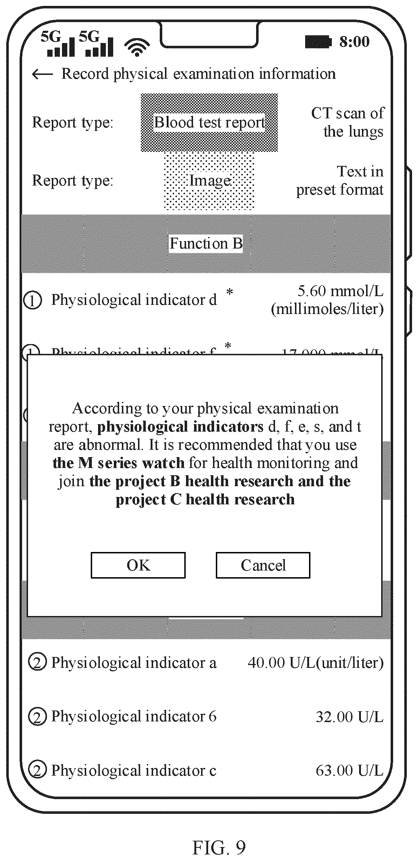

As described in the foregoing example, the mobile phone generates recommendation information based on the abnormal physiological indicator information of the user, and displays the recommendation information in an interface shown in FIG. 9. In this example, the recommended information is text information. For example, the text information is "According to your physical examination report, physiological indicators d, f, e, s, and t are abnormal. It is recommended that you use the M series watch for health detection and join the project B health research and the project C health research".

It should be noted that, after the second device generates the recommendation information, the second device needs to perform different processing based on different operations performed by the user on the recommendation information. When the user performs a first operation on the recommendation information, the second device performs the following step S508a; when the user performs a second operation on the recommendation information, the second device performs the following step S508b; when the user performs a third operation on the recommendation information, the second device performs the following step S508c; and when the user performs a fourth operation on the recommendation information, the second device performs the following step S508d. Details are as follows.

Step S508a: In response to the first operation of the user on the recommendation information, the second device detects whether the user has a physiological data detection device.

In this embodiment of this application, the first operation of the user on the recommendation information may be that the user indicates the second device to continue to perform a next step. In this case, if the user performs the first operation on the recommendation information, it indicates that the user needs the second device to perform next detection. Because the physiological indicator information of the user has been displayed in the foregoing steps, when performing the next step, the second device may enter a procedure of detecting a target physiological indicator. When the target physiological indicator is detected, data detected in the physiological data detection device needs to be used. Therefore, the second device needs to first detect whether the user has the physiological data detection device. In this case, the second device may detect whether the user has the physiological data detection device by detecting whether the second device has a record of a link to the physiological data detection device. If the second device records the record of the link to the physiological data detection device, it is determined that the user has the physiological data detection device.

The following steps performed by the second device vary according to different determining results of the second device. When it is determined that the user does not have the physiological data detection device, step S509a is performed. When it is determined that the user has the physiological data detection device, step S509b is performed.

Step S508b: The second device outputs information about the abnormal physiological indicator of the user in response to the second operation of the user on the recommendation information.

In this embodiment of this application, the second operation of the user on the recommendation information may be an operation of indicating, by the user, the second device to output the detailed information about the abnormal physiological indicator. In this case, when the user performs the second operation on the recommendation information, for example, the user taps the abnormal physiological indicator in the recommendation information, the second device obtains and outputs the detailed information about the abnormal physiological indicator in response to the second operation. For the obtaining and outputting, by the second device, the detailed information about the abnormal physiological indicator, refer to step S506. Details are not described herein again.

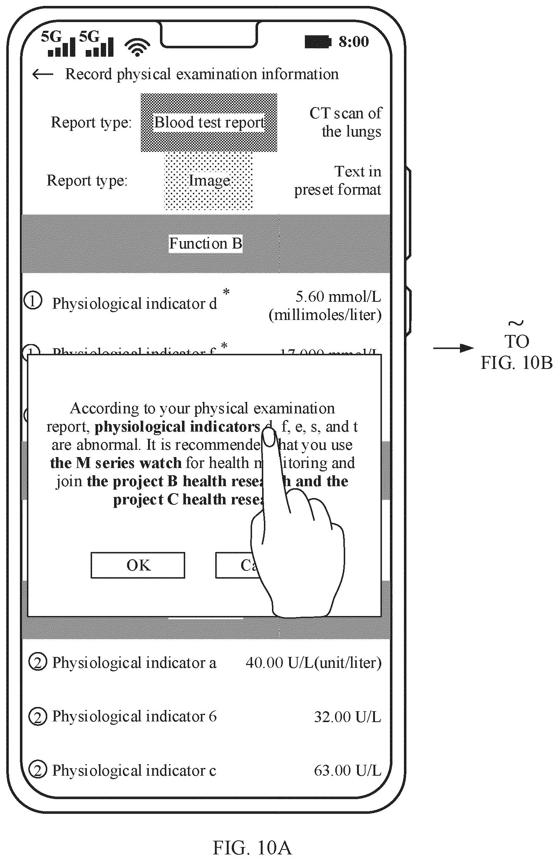

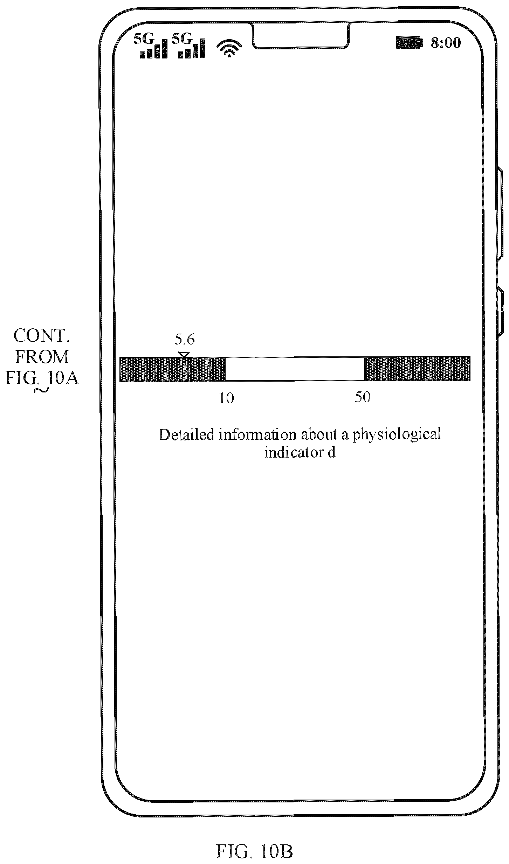

As shown in the foregoing example, the interface displayed in FIG. 9 includes the recommendation information. As shown in FIG. 10A and FIG. 10B, the user taps the abnormal physiological indicator d, f, e, s, or t in the recommendation information, and the mobile phone displays detailed information about the abnormal physiological indicator d, f, e, s, or t. It is assumed that the user taps the abnormal physiological indicator d, the mobile phone displays the detailed information about the physiological indicator d.

Step S508c: In response to the third operation of the user on the recommendation information, the second device outputs the information about the physiological data detection device capable of detecting the abnormal physiological indicator of the user.

In this embodiment of this application, the third operation of the user on the recommendation information may be an operation of indicating, by the user, the second device to display information about the physiological data detection device. When the user performs the third operation on the recommendation information, for example, the user taps the physiological data detection device in the recommendation information, the second device may obtain the information about the physiological data detection device from the network or another device in response to the third operation, and output the obtained information about the physiological data detection device. A manner in which the second device outputs the information about the physiological data detection device capable of detecting the abnormal physiological indicator of the user may be displayed through the display device, or may be played in a voice broadcast manner, or may be output in another manner. This is not limited in this application.

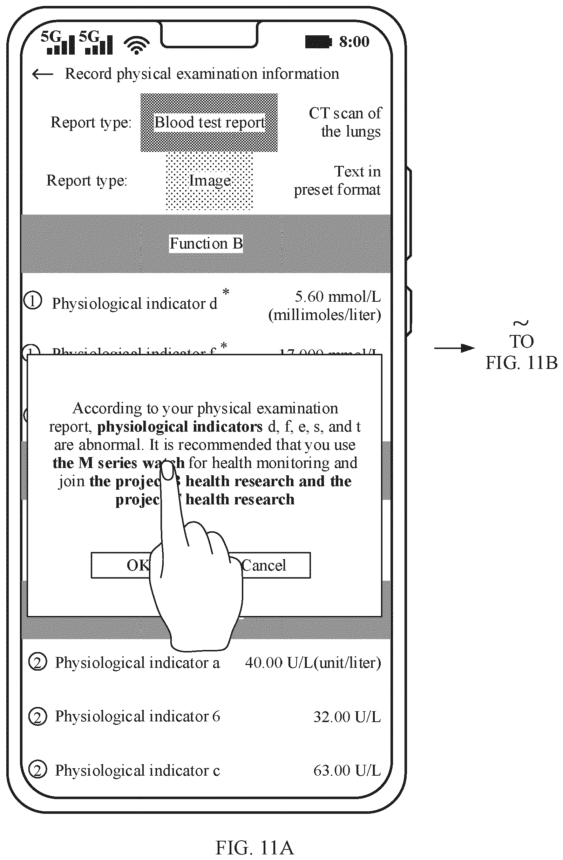

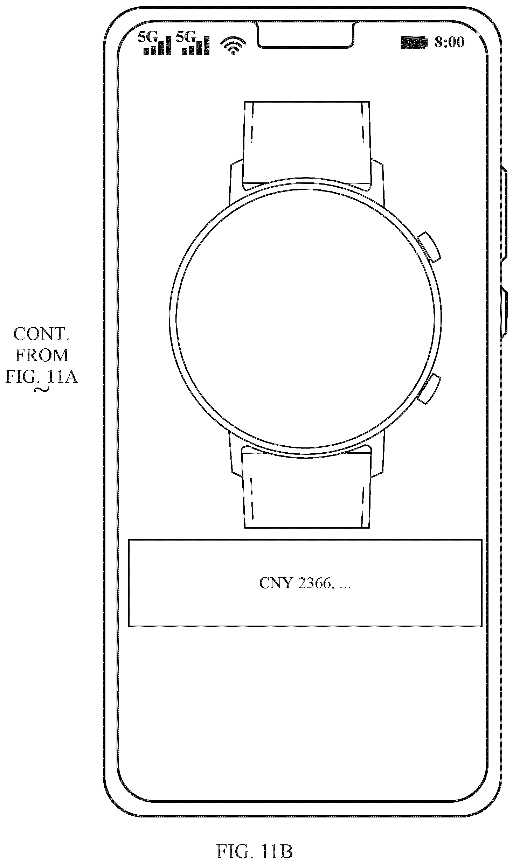

As shown in the foregoing example, the interface displayed in FIG. 9 includes the recommendation information. As shown in FIG. 11A and FIG. 11B, after the user taps the physiological data detection device in the recommendation information, for example, an M series watch, the mobile phone may obtain information about the M series watch from the network, for example, introduction information or purchase information of the M series watch, and the mobile phone displays the information about the M series watch on a display interface.

Step S508d: The second device outputs the medical research information related to the abnormal physiological indicator of the user in response to the fourth operation of the user on the recommendation information.

In this embodiment of this application, the fourth operation of the user on the recommendation information may be that the user indicates the second device to display the medical research project information related to the abnormal physiological indicator. When the user performs the fourth operation on the recommendation information, for example, the user taps the medical research information, the second device may obtain, in response to the fourth operation, the medical research information related to the abnormal physiological indicator of the user from the network or another device, and output the obtained medical research information related to the abnormal physiological indicator.

Medical research information on different physiological indicators is related information of medical researches performed on the different physiological indicators, and includes a normal value range of the physiological indicator, an improvement suggestion, analysis information of the physiological indicator data information of the user, and the like.

In some embodiments, the second device may further display other medical research information. In this case, in response to a display operation of the user for the medical research information, the second device determines a display priority of each piece of the medical research information, and outputs a plurality of pieces of the medical research information based on display priorities.

Medical research information related to the abnormal physiological indicator of the user has a highest display priority.

In other words, if the user needs to determine which medical research information that the user can join by using the second device, the user may perform a display operation on the medical research information in the second device. In this case, when receiving the display operation on the medical research information performed by the user, the second device may determine a display priority of each piece of the medical research information that the user can join in the second device, and output the medical research information based on the priority of each piece of the medical research information.

In some embodiments, the second device may set a display priority of the medical research information related to the abnormal physiological indicator of the user to a highest priority. In this way, the medical research information related to the abnormal physiological indicator of the user is first displayed in the displayed medical research information. In some embodiments, when determining a display priority of other medical research information, the second device may determine the display priority of the other medical research information based on a use frequency of the other medical research information. In this way, medical research information with a higher use frequency has a higher display priority, but is lower than the display priority of the medical research information related to the abnormal physiological indicator of the user.

As shown in the foregoing example, the interface displayed in FIG. 9 includes the recommendation information. As shown in FIG. 12a-1 and FIG. 12a-2, after the user taps the medical research information in the recommendation information, for example, the project B health research, the mobile phone may obtain related information of the liver fat research from the network or another device, for example, introduction information of the project B, and the mobile phone displays related information of the project B health research on a display interface. In the interface shown in FIG. 12a-1 and FIG. 12a-2, the interface includes a home page control 1201. In this case, in response to the user tapping the home page control 1201, the mobile phone displays an interface shown in FIG. 12b-1 and FIG. 12b-2. The interface is a home page of the medical research information, and includes all medical research information that the user can join by using the second device, and a display sequence of all the medical research information is determined by the second device. The display priority of the medical research information related to the abnormal physiological indicator of the user is higher than a display priority of the other medical research information. To be specific, in the interface shown in FIG. 12b-1 and FIG. 12b-2, when there are two pieces of medical research information related to the abnormal physiological indicator of the user, the first two pieces of medical research information in the interface shown in FIG. 12b-1 and FIG. 12b-2 are the medical research information related to the abnormal physiological indicator of the user.

Step S509a: When it is detected that the user does not have the physiological data detection device, the second device outputs information about a physiological data detection device capable of detecting the abnormal physiological indicator of the user.

In this embodiment of this application, because the physiological indicator of the user is detected, the physiological data detection device needs to be used to detect a daily physiological indicator of the user. When the second device detects that the user does not have the physiological data detection device, the user cannot continue to perform a physiological indicator detection step. In this case, the second device may recommend, to the user based on the abnormal physiological indicator information of the user, the physiological data detection device capable of detecting the abnormal physiological indicator of the user, that is, output the physiological data detection device capable of detecting the abnormal physiological indicator of the user, for the user to purchase the physiological data detection device capable of detecting the abnormal physiological indicator of the user.

Step S509b: When it is detected that the user has the physiological data detection device, the second device displays the abnormal physiological indicator information in a preset sequence.

In this embodiment of this application, after detecting that the user has the physiological data detection device, the second device may sort and display abnormal physiological indicators of the user in the preset sequence, so that the user determines, from the abnormal physiological indicators, a physiological indicator that needs to be detected. In some embodiments, the second device may sort and display the abnormal physiological indicator information in descending order based on severity of the abnormal physiological indicators.

As shown in the foregoing example, the interface displayed in FIG. 9 includes the recommendation information. As shown in FIG. 13A and FIG. 13B, the user taps an "OK" control in the recommendation information, and the mobile phone may further detect whether a record of a link to the physiological data detection device is stored in the mobile phone. If the record of the link to the physiological data detection device is not stored in the mobile phone, the physiological data detection device capable of detecting the abnormal physiological indicator of the user may be determined, and information about the physiological data detection device capable of detecting the abnormal physiological indicator of the user is displayed, as shown in FIG. 11A and FIG. 11B. If the record of the link to the physiological data detection device is stored the mobile phone, the mobile phone may determine that the user has the physiological data detection device. In this case, the mobile phone may sort abnormal physiological indicators in descending order based on severity of the abnormal physiological indicators of the user, and display the sorted abnormal physiological indicators, as shown in an interface in FIG. 13A and FIG. 13B.

Step S510: The second device determines a target physiological indicator of the user in response to a selection operation of the user on the abnormal physiological indicator information in the abnormal physiological indicator information displayed in the preset sequence.

In this embodiment of this application, the user may detect the abnormal physiological indicator, to learn whether a daily living habit of the user can improve the abnormal physiological indicator. In this case, after the second device displays the abnormal physiological indicator information in the preset sequence, the user may select an abnormal physiological indicator from the abnormal physiological indicator information displayed by the second device as the to-be-detected physiological indicator. In this case, the second device determines, in response to the selection operation of the user on the abnormal physiological indicator information, the abnormal physiological indicator selected by the user as the target physiological indicator of the user.

In some embodiments, when the user selects a plurality of abnormal physiological indicators and at least two of the plurality of abnormal physiological indicators belong to physiological indicators for a same device, the at least two abnormal physiological indicators may be classified into a same type of physiological indicator, and the type of physiological indicator is referred to as the target physiological indicator.

Step S511: The second device detects whether the physiological data detection device of the user includes a fourth device.

The fourth device is a physiological data detection device capable of detecting the target physiological indicator of the user.

In this embodiment of this application, after the second device obtains the target physiological indicator, because a case in which a physiological indicator detected by the physiological data detection device of the user does not include the target physiological indicator may exist, the second device needs to further detect whether the user has the fourth device capable of detecting the target physiological indicator. In this case, the second device may obtain a detection function of the physiological data detection device of the user, and learn, based on the detection function of the physiological data detection device, whether the physiological data detection device of the user includes the fourth device.

It should be noted that the second device performs different steps according to different detection results. When the second device detects that the physiological data detection device of the user does not include the fourth device, the following step S512a is performed. When the second device detects that the physiological data detection device of the user includes the fourth device, the following step S512b is performed.

Step S512a: When it is detected that the physiological data detection device of the user does not include the fourth device, the second device outputs information about the fourth device.

In this embodiment of this application, when the second device detects that the physiological data detection device of the user does not include the fourth device, it indicates that the physiological data detection data of the user cannot detect the target physiological indicator of the user. In this case, the second device cannot predict whether the target physiological indicator can be improved based on the living habit of the user. Therefore, the second device may recommend, to the user, the fourth device capable of detecting the target physiological indicator of the user, for the user to purchase the fourth device capable of detecting the target physiological indicator of the user. In some embodiments, the second device may obtain information about the fourth device through the network or another device, and the second device may display the information about the fourth device in a display interface in a display manner. Alternatively, the information about the fourth device is played to the user in a voice broadcast manner. Certainly, the second device may alternatively send the information about the fourth device to the user in another manner. This is not limited in this application.

Step S512b: When the physiological data detection device of the user includes the fourth device, the second device sends the target physiological indicator of the user to the first device. The first device receives the target physiological indicator of the user sent by the second device.

In this embodiment of this application, when the second device detects that the physiological data detection device of the user includes the fourth device, it indicates that the user may perform routine detection on the target physiological indicator of the user by using the fourth device. In this case, the second device sends the target physiological indicator to the first device. The first device receives the target physiological indicator of the user. For details, refer to step S402 and step S101. Details are not described herein again.

Step S513: The first device obtains a prediction model for the target physiological indicator based on the target physiological indicator.

For details, refer to step S102. Details are not described herein again.

In a possible implementation, that a target reference user is determined based on the target physiological indicator from a user whose physiological indicator data information, living data information, and physiological data information are obtained includes:
determining, based on the target physiological indicator, the target reference user from a user that is in the reference user and whose physiological indicator data information, living data information, and physiological data information are obtained.

In other words, when obtaining the target physiological indicator and needing to construct a prediction mode of the target physiological indicator, the first device needs to first determine data information used to train the network model. In this case, the first device needs to determine the target reference user corresponding to the target physiological indicator from the user that is in the reference user determined in step S504 and whose physiological indicator data information, living data information, and physiological data information are obtained. Obtained physiological indicator data information of the target reference user includes the target physiological indicator. In other words, in step S504, reference users of the user are determined for different abnormal physiological indicators. In this step, the reference user of the target physiological indicator may be determined based on the target physiological indicator, and the target reference user is determined from the user that is in the reference user of the target physiological indicator and whose physiological indicator data information, living data information, and physiological data information are obtained.

In this way, a time for determining the target reference user can be shortened, a training time of the prediction model can be reduced, and prediction efficiency of the target physiological indicator can be improved.

Step S514: The second device obtains the living data information and the physiological data information of the user, and sends the living data information and the physiological data information of the user to the first device. The first device receives the living data information and the physiological data information of the user sent by the second device.

For details, refer to step S403 and step S103. Details are not described herein again.

Step S515: The first device inputs the living data information and the physiological data information of the user into the prediction model, to obtain a prediction result output by the prediction model.

For details, refer to step S103. Details are not described herein again.

Step S516: The first device determines a positive reference user from the target reference user.

The positive reference user is a user whose target physiological indicator is improved.

In this embodiment of this application, the first device determines, from the target reference user, a user whose target physiological indicator is improved as the positive reference user. In other words, the first device determines a target reference user whose target physiological indicator is improved as the positive reference user.

Step S517: The first device generates improvement suggestion information for the target physiological indicator based on living data information and physiological data information of the positive reference user.

In this embodiment of this application, the first device may generate the improvement suggestion information for the target physiological indicator based on the obtained living data information and the obtained physiological data information of the positive reference user. For example, the improvement suggestion information for the target physiological indicator is generated based on daily diet information, exercise information, and the like of the positive reference user.

Step S518: The first device sends the improvement suggestion information for the target physiological indicator and the prediction result to the second device. The second device receives the improvement suggestion information and the prediction result sent by the first device.

In this embodiment of this application, after generating the improvement suggestion information for the target physiological indicator and obtaining the prediction result, the first device may send the improvement suggestion information for the target physiological indicator and the prediction result to the second device, so that the second device sends the improvement suggestion information and the prediction result to the user. The second device receives the improvement suggestion information and the prediction result sent by the first device.

Step S519: The second device outputs the prediction result and the improvement suggestion information.

In this embodiment of this application, after receiving the improvement suggestion information and the prediction result sent by the first device, the second device may output the prediction result and the improvement suggestion information in a display manner, a voice broadcast manner, or another manner, for the user to learn the prediction result and the improvement suggestion information.

FIG. 14A to FIG. 14D are a schematic flowchart of a health management method according to an embodiment of this application. The first device may be a cloud server, or may be an electronic device that can directly interact with a user. In this embodiment of this application, an example in which the first device is an electronic device that can directly interact with a user is used for description. As shown in FIG. 14A to FIG. 14D, the method includes the following steps.

Step S1401: In response to a physical examination information input operation of a user, a first device performs recognition processing on physical examination information input by the user, to obtain physiological indicator data information of the user.

For details, refer to step S501. Details are not described herein again.

Step S1402: The first device sends the physiological indicator data information of the user to a third device. The third device receives the physiological indicator data information of the user sent by the first device.

The third device is a cloud server.

For details, refer to step S502. Details are not described herein again.

Step S1403: The third device obtains physiological indicator reference information of the user, and determines abnormal physiological indicator information and normal physiological indicator information of the user based on the physiological indicator reference information of the user and the physiological indicator data information of the user.

For details, refer to step S503. Details are not described herein again.

Step S1404: The third device determines, based on the abnormal physiological indicator information of the user, a reference user for the user from another user whose physiological indicator data information is obtained.

For details, refer to step S504. Details are not described herein again.

Step S1405: The third device sends the abnormal physiological indicator information and the normal physiological indicator information of the user to the first device. The first device receives the abnormal physiological indicator information and the normal physiological indicator information of the user sent by the third device.

For details, refer to step S505. Details are not described herein again.

Step S1406: The first device outputs the abnormal physiological indicator information and the normal physiological indicator information of the user.

For details, refer to step S506. Details are not described herein again.

Step S1407: The first device generates recommendation information based on the abnormal physiological indicator information of the user and displays the recommendation information.

For details, refer to step S507. Details are not described herein again.

Step S1408a: In response to a first operation of the user on the recommendation information, the first device detects whether the user has a physiological data detection device.

For details, refer to step S508a. Details are not described herein again.

Step S1408b: The first device outputs information about the abnormal physiological indicator of the user in response to a second operation of the user on the recommendation information.

For details, refer to step S508b. Details are not described herein again.

Step S1408c: In response to a third operation of the user on the recommendation information, the first device outputs information about a physiological data detection device capable of detecting the abnormal physiological indicator of the user.

For details, refer to step S508c. Details are not described herein again.

Step S1408d: The first device outputs medical research information on the abnormal physiological indicator of the user in response to a fourth operation of the user on the recommendation information.

For details, refer to step S508d. Details are not described herein again.

Step S1409a: When it is detected that the user does not have the physiological data detection device, the first device outputs information about a physiological data detection device capable of detecting the abnormal physiological indicator of the user.

For details, refer to step S509a. Details are not described herein again.

Step S1409b: When it is detected that the user has the physiological data detection device, a second device displays the abnormal physiological indicator information in a preset sequence.

For details, refer to step S509b. Details are not described herein again.

Step S1410: The second device determines a target physiological indicator of the user in response to a selection operation of the user on the abnormal physiological indicator information in the abnormal physiological indicator information displayed in the preset sequence.

For details, refer to step S510. Details are not described herein again.

Step S1411: The first device detects whether the physiological data detection device of the user includes a fourth device.

For details, refer to step S511. Details are not described herein again.

Step S1412a: When it is detected that the physiological data detection device of the user does not include the fourth device, the first device outputs information about the fourth device.

For details, refer to step S512a. Details are not described herein again.

Step S1412b: When the physiological data detection device of the user includes the fourth device, the first device sends the target physiological indicator of the user to the third device. The third device receives the target physiological indicator of the user sent by the first device.

For details, refer to step S512b. Details are not described herein again.

Step S1413: The third device obtains a prediction model for the target physiological indicator based on the target physiological indicator.

For details, refer to step S513. Details are not described herein again.

Step S1414: The third device sends the prediction model to the first device, and the first device receives the prediction model that is for the target physiological indicator and that is sent by the third device.

For details, refer to step S101. Details are not described herein again.

Step S1415: The first device obtains living data information and physiological data information of the user, and inputs the living data information and the physiological data information of the user into the prediction model, to obtain a prediction result output by the prediction model.

For details, refer to step S103. Details are not described herein again.

Step S1416: The third device determines a positive reference user from a target reference user.

The positive reference user is a user whose target physiological indicator is improved.

For details, refer to step S516. Details are not described herein again.

Step S1417: The third device generates improvement suggestion information for the target physiological indicator based on living data information and physiological data information of the positive reference user.

For details, refer to step S517. Details are not described herein again.

Step S1418: The third device sends the improvement suggestion information for the target physiological indicator to the first device. The first device receives the improvement suggestion information sent by the third device.

For details, refer to step S518. Details are not described herein again.

Step S1419: The first device outputs the prediction result and the improvement suggestion information.

For details, refer to step S519. Details are not described herein again.

FIG. 15a is a diagram of a structure of a health management apparatus according to an embodiment of this application. As shown in FIG. 15a, the health management apparatus includes:
an obtaining unit 1501, configured to determine a target physiological indicator of a user, where
the obtaining unit 1501 is further configured to obtain a prediction model for the target physiological indicator based on the target physiological indicator; and
a processing unit 1502, configured to obtain living data information and physiological data information of the user, and input the living data information and the physiological data information of the user into the prediction model, to obtain a prediction result output by the prediction model.

The prediction result represents a predicted impact of the living data information and the physiological data information of the user on a change trend of the target physiological indicator, and the physiological data information includes related data of the target physiological indicator.

In a possible implementation, as shown in FIG. 15b, the health management apparatus further includes:
an output unit 1503, configured to output the prediction result.

In a possible implementation, the obtaining unit 1501 is specifically configured to determine, based on the target physiological indicator, a target reference user from a user whose physiological indicator data information, living data information, and physiological data information are obtained. The prediction model is constructed based on obtained physiological indicator data information, obtained living data information, and obtained physiological data information of the target reference user.

The obtained physiological indicator data information of the target reference user includes data information of the target physiological indicator.

Alternatively, the obtaining unit 1501 is specifically configured to: send the target physiological indicator to the third device; and receive the prediction model that is for the target physiological indicator and that is sent by a third device.

In a possible implementation, the obtaining unit 1501 is specifically configured to receive the target physiological indicator of the user sent by a second device.

Alternatively, the obtaining unit 1501 is specifically configured to determine the target physiological indicator of the user in response to a selection operation of the user.

In a possible implementation, the processing unit 1502 is specifically configured to receive the living data information and the physiological data information of the user sent by the second device.

Alternatively, the processing unit 1502 is specifically configured to obtain the living data information of the user in response to an input operation of the living data information of the user. The physiological data information of the user detected by a fourth device is obtained from the fourth device.

In a possible implementation, the sending unit 1503 is specifically configured to send the prediction result to the second device.

In a possible implementation, the obtaining unit 1501 is further configured to receive physiological indicator data information in a physical examination report of the user sent by the second device.

The processing unit 1502 is further configured to: obtain physiological indicator reference information of the user, and determine abnormal physiological indicator information and normal physiological indicator information of the user based on the physiological indicator reference information of the user and the physiological indicator data information of the user.

The sending unit 1503 is further configured to send the abnormal physiological indicator information and the normal physiological indicator information of the user to the second device.

In a possible implementation, the processing unit 1502 is further configured to determine, based on the abnormal physiological indicator information of the user, a reference user for the user from another user whose physiological indicator data information is obtained.

The processing unit 1502 is specifically configured to determine, based on the target physiological indicator, the target reference user from a user that is in the reference user and whose physiological indicator data information, living data information, and physiological data information are obtained.

In a possible implementation, the processing unit 1502 is further configured to: determine a positive reference user from the target reference user; and generate improvement suggestion information for the target physiological indicator based on living data information and physiological data information of the positive reference user. The positive reference user is a user whose target physiological indicator is improved.

The output unit 1503 is specifically configured to output the prediction result and the improvement suggestion information.

In a possible implementation, the obtaining unit 1501 is further configured to obtain abnormal physiological indicator information and normal physiological indicator information of the user in response to a physical examination information input operation of the user.

The output unit 1503 is further configured to output the abnormal physiological indicator information and the normal physiological indicator information of the user.

In a possible implementation, the processing unit 1502 is further configured to: detect whether the user has a physiological data detection device; and when it is detected that the user has the physiological data detection device, display the abnormal physiological indicator information in a preset sequence.

The physiological data detection device is a device for detecting at least one physiological indicator of the user.

The obtaining unit 1501 is specifically configured to determine the target physiological indicator of the user in response to a selection operation of the user on the abnormal physiological indicator information in the abnormal physiological indicator information displayed in the preset sequence.

In a possible implementation, the processing unit 1502 is further configured to: when it is detected that the user does not have the physiological data detection device, output information about a physiological data detection device capable of detecting the abnormal physiological indicator of the user.

In a possible implementation, the processing unit 1502 is further configured to detect whether physiological data detection device of the user includes a fourth device.

The fourth device is a physiological data detection device capable of detecting the target physiological indicator of the user.

The obtaining unit 1501 is specifically configured to: when the physiological data detection device of the user includes the fourth device, obtain the prediction model for the target physiological indicator based on the target physiological indicator.

In a possible implementation, the processing unit 1502 is further configured to: when it is detected that the physiological data detection device of the user does not include the fourth device, output information about the fourth device.

In a possible implementation, the processing unit 1502 is further configured to generate recommendation information based on the abnormal physiological indicator information of the user, and display the recommendation information.

The recommendation information includes the information about the physiological data detection device capable of detecting the abnormal physiological indicator of the user, and medical research information related to the abnormal physiological indicator of the user.

In a possible implementation, the recommendation information further includes the abnormal physiological indicator of the user.

In this case, the processing unit 1502 is specifically configured to: in response to a first operation of the user on the recommendation information, detect whether the user has the physiological data detection device.

In a possible implementation, the processing unit 1502 is further configured to: output information about the abnormal physiological indicator of the user in response to a second operation of the user on the recommendation information; or
in response to a third operation of the user on the recommendation information, output the information about the physiological data detection device capable of detecting the abnormal physiological indicator of the user; or
output the medical research information related to the abnormal physiological indicator of the user in response to a fourth operation of the user on the recommendation information.

In a possible implementation, in response to a display operation of the user on the medical research information, the processing unit 1502 is further configured to determine a display priority of each piece of the medical research information, and display a plurality of pieces of the medical research information based on display priorities.

Medical research information related to the abnormal physiological indicator of the user has a highest display priority.

In a possible implementation, the obtaining unit 1501 is further configured to receive improvement suggestion information sent by the third device.

FIG. 16 is a diagram of a structure of a health management apparatus according to an embodiment of this application. As shown in FIG. 16, the health management apparatus includes:
an obtaining unit 1601, configured to determine a target physiological indicator of a user in response to a selection operation of the user; and
a sending unit 1602, configured to send the target physiological indicator of the user to a first device.

The obtaining unit 1601 is further configured to obtain living data information and physiological data information of the user.

The sending unit 1602 is further configured to send the living data information and the physiological data information of the user to the first device.

In a possible implementation, as shown in FIG. 17, the health management apparatus further includes:
a receiving unit 1603, configured to receive a prediction result sent by the first device.

The sending unit 1602 is further configured to output the prediction result.

In a possible implementation, the obtaining unit 1601 is specifically configured to: obtain the living data information of the user in response to an input operation of the living data information of the user; and obtain, from a fourth device, the physiological data information of the user detected by the fourth device.

In a possible implementation, as shown in FIG. 17, the health management apparatus further includes:
a processing unit 1604, configured to: in response to a physical examination information input operation of the user, perform recognition processing on physical examination information input by the user, to obtain physiological indicator data information of the user.

The sending unit 1602 is further configured to send the physiological indicator data information of the user to the first device.

The receiving unit 1603 is further configured to receive abnormal physiological indicator information and normal physiological indicator information of the user sent by the first device.

The sending unit 1602 is further configured to output the abnormal physiological indicator information and the normal physiological indicator information of the user.

In a possible implementation, the processing unit 1604 is further configured to: detect whether the user has a physiological data detection device; and when it is detected that the user has the physiological data detection device, display the abnormal physiological indicator information in a preset sequence.

The physiological data detection device is a device for detecting at least one physiological indicator of the user.

The obtaining unit 1601 is specifically configured to determine the target physiological indicator of the user in response to a selection operation of the user on the abnormal physiological indicator information in the abnormal physiological indicator information displayed in the preset sequence.

In a possible implementation, the processing unit 1604 is further configured to: when it is detected that the user does not have the physiological data detection device, output information about a physiological data detection device capable of detecting the abnormal physiological indicator of the user.

In a possible implementation, the processing unit 1604 is further configured to detect whether physiological data detection device of the user includes a fourth device.

The fourth device is a physiological data detection device capable of detecting the target physiological indicator of the user.

The sending unit 1602 is specifically configured to: when the physiological data detection device of the user includes the fourth device, send the target physiological indicator of the user to the first device.

In a possible implementation, the processing unit 1604 is further configured to: when it is detected that the physiological data detection device of the user does not include the fourth device, output information about the fourth device.

In a possible implementation, the processing unit 1604 is further configured to generate recommendation information based on the abnormal physiological indicator information of the user, and display the recommendation information.

The recommendation information includes at least one of the information about the physiological data detection device capable of detecting the abnormal physiological indicator of the user and medical research information related to the abnormal physiological indicator of the user.

In a possible implementation, the recommendation information further includes the abnormal physiological indicator of the user.

The processing unit 1604 is specifically configured to: in response to a first operation of the user on the recommendation information, detect whether the user has the physiological data detection device.

In a possible implementation, the processing unit 1604 is further configured to: output information about the abnormal physiological indicator of the user in response to a second operation of the user on the recommendation information; or
in response to a third operation of the user on the recommendation information, output the information about the physiological data detection device capable of detecting the abnormal physiological indicator of the user; or
output the medical research information related to the abnormal physiological indicator of the user in response to a fourth operation of the user on the recommendation information.

In a possible implementation, in response to a display operation of the user on the medical research information, the processing unit 1604 is further configured to determine a display priority of each piece of the medical research information, and display a plurality of pieces of the medical research information based on display priorities.

Medical research information related to the abnormal physiological indicator of the user has a highest display priority.

In a possible implementation, the receiving unit 1603 is further configured to receive improvement suggestion information sent by the first device.

The sending unit 1602 is specifically configured to output the prediction result and the improvement suggestion information.

In correspondence to the foregoing embodiments, this application further provides an electronic device. FIG. 18 is a diagram of a structure of an electronic device according to an embodiment of the present invention. An electronic device 1800 may include a processor 1801, a memory 1802, and a communication unit 1803. These components communicate with each other through one or more buses. A person skilled in the art may understand that a structure of a server shown in the figure does not constitute a limitation on embodiments of the present invention. The structure may be a bus structure, or may be a star structure, or may further include more or fewer components than those shown in the figure, or combine some components, or have different component arrangements.

The communication unit 1803 is configured to: establish a communication channel, so that a storage device can communicate with another device; and receive user data sent by the another device or send user data to the another device.

The processor 1801 is a control center of the storage device, connects to various parts of the entire electronic device through various interfaces and lines, and performs various functions of the electronic device and/or data processing by running or executing a software program and/or a module stored in the memory 1802 and invoking data stored in the memory. The processor may include an integrated circuit (Integrated Circuit, IC), for example, may include a single packaged IC, or may include a plurality of connected packaged ICs with a same function or different functions. For example, the processor 1801 may include only a central processing unit (central processing unit, CPU). In this implementation of the present invention, the CPU may be a single computing core, or may include a plurality of computing cores.

The memory 1802 is configured to store execution instructions of the processor 1801. The memory 1802 may be implemented by any type of a volatile or nonvolatile storage device or a combination thereof, for example, a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic disk, or an optical disc.

When the execution instructions in the memory 1802 are executed by the processor 1801, the electronic device 1800 is enabled to perform some or all steps in the embodiment shown in FIG. 5A to FIG. 5D and the embodiment shown in FIG. 14A to FIG. 14D.

In a specific implementation, the present invention further provides a computer storage medium. The computer storage medium may store a program, and when the program is executed, some or all of steps of embodiments of the health management method provided in the present invention may be performed. The storage medium may be a magnetic disk, an optical disc, a read-only memory (read-only memory, ROM), a random access memory (random access memory, RAM), or the like.

A person skilled in the art may clearly understand that, the technologies in embodiments of the present invention may be implemented by software in addition to a necessary general hardware platform. Based on such an understanding, the technical solutions of embodiments of the present invention essentially or the part contributing to the conventional technology may be implemented in a form of a software product. The computer software product may be stored in a storage medium, such as a ROM/RAM, a hard disk, or an optical disc, and includes several instructions for instructing a computer device (which may be a personal computer, a server, or a network device) to perform the methods described in embodiments or some parts of embodiments of the present invention.

Same or similar parts of embodiments of this specification may be referred to each other. Especially, apparatus embodiments and terminal embodiments are basically similar to method embodiments, and therefore are described briefly. For related parts, refer to descriptions in the method embodiments.

## Claims

1. A health management method, applied to a first device, wherein the method comprises:
determining a target physiological indicator of a user; and
obtaining living data information and physiological data information of the user, and inputting the living data information and the physiological data information of the user into a prediction model, to obtain a prediction result output by the prediction model, wherein the prediction result represents a predicted impact of the living data information and the physiological data information of the user on a change of the target physiological indicator.

2. The method according to claim 1, further comprising:
determining, based on the target physiological indicator, a target reference user from a user whose physiological indicator data information, living data information, and physiological data information are obtained, wherein obtained physiological indicator data information of the target reference user comprises data information of the target physiological indicator; and
constructing the prediction model based on the obtained physiological indicator data information, obtained living data information, and obtained physiological data information of the target reference user.

3. The method according to claim 2, wherein the determining a target physiological indicator of a user comprises:
receiving the target physiological indicator of the user sent by a second device; and
the obtaining living data information and physiological data information of the user comprises:
receiving the living data information and the physiological data information of the user sent by the second device.

4. The method according to claim 2 or 3, further comprising:
receiving physiological indicator data information in a physical examination report of the user sent by the second device;
obtaining physiological indicator reference information of the user, and determining abnormal physiological indicator information and normal physiological indicator information of the user based on the physiological indicator reference information of the user and the physiological indicator data information of the user; and
sending the abnormal physiological indicator information and the normal physiological indicator information of the user to the second device.

5. The method according to claim 4, wherein the method further comprises:
determining, based on the abnormal physiological indicator information of the user, a reference user for the user from another user whose physiological indicator data information is obtained; and
the determining, based on the target physiological indicator, a target reference user from a user whose physiological indicator data information, living data information, and physiological data information are obtained comprises:
determining, based on the target physiological indicator, the target reference user from a user that is in the reference user and whose physiological indicator data information, living data information, and physiological data information are obtained.

6. The method according to any one of claims 2 to 5, further comprising:
determining a positive reference user from the target reference user, wherein the positive reference user is a user whose target physiological indicator is improved; and
generating improvement suggestion information for the target physiological indicator based on living data information and physiological data information of the positive reference user.

7. The method according to claim 1, wherein the determining a target physiological indicator of a user comprises:
determining the target physiological indicator of the user in response to a selection operation of the user.

8. The method according to claim 7, further comprising:
sending the target physiological indicator to a third device; and
receiving the prediction model that is for the target physiological indicator and that is sent by the third device.

9. The method according to claim 7, wherein the obtaining living data information of the user comprises:
obtaining the living data information of the user in response to an input operation of the living data information of the user.

10. The method according to claim 7, wherein the obtaining physiological data information of the user comprises:
obtaining, from a fourth device, the physiological data information of the user detected by the fourth device.

11. The method according to claim 10, further comprising:
obtaining abnormal physiological indicator information and normal physiological indicator information of the user in response to a physical examination information input operation of the user; and
outputting the abnormal physiological indicator information and the normal physiological indicator information of the user.

12. The method according to claim 11, wherein the method further comprises:
detecting whether the user has a physiological data detection device, wherein the physiological data detection device is a device for detecting at least one physiological indicator of the user; and
when it is detected that the user has the physiological data detection device, displaying the abnormal physiological indicator information in a preset sequence; and
the determining the target physiological indicator of the user in response to a selection operation of the user comprises:
determining the target physiological indicator of the user in response to the selection operation of the user on the abnormal physiological indicator information in the abnormal physiological indicator information displayed in the preset sequence.

13. The method according to claim 12, further comprising:
when it is detected that the user does not have the physiological data detection device, outputting information about a physiological data detection device capable of detecting an abnormal physiological indicator of the user.

14. The method according to claim 12, wherein the method further comprises:
detecting whether the physiological data detection device of the user comprises a fourth device, wherein the fourth device is a physiological data detection device capable of detecting the target physiological indicator of the user; and
the obtaining the prediction model for the target physiological indicator based on the target physiological indicator comprises:
when the physiological data detection device of the user comprises the fourth device, obtaining the prediction model for the target physiological indicator based on the target physiological indicator.

15. The method according to claim 14, further comprising:
when it is detected that the physiological data detection device of the user does not comprise the fourth device, outputting information about the fourth device.

16. The method according to any one of claims 12 to 15, wherein before the detecting whether the user has a physiological data detection device, the method further comprises:
generating recommendation information based on the abnormal physiological indicator information of the user and displaying the recommendation information, wherein the recommendation information comprises the information about the physiological data detection device capable of detecting the abnormal physiological indicator of the user and medical research information related to the abnormal physiological indicator of the user; and
the detecting whether the user has a physiological data detection device comprises:
in response to a first operation of the user on the recommendation information, detecting whether the user has the physiological data detection device.

17. The method according to claim 16, further comprising:
outputting information about the abnormal physiological indicator of the user in response to a second operation of the user on the recommendation information; or
in response to a third operation of the user on the recommendation information, outputting the information about the physiological data detection device capable of detecting the abnormal physiological indicator of the user; or
outputting the medical research information related to the abnormal physiological indicator of the user in response to a fourth operation of the user on the recommendation information.

18. The method according to any one of claims 11 to 16, further comprising:
in response to a display operation of the user for medical research information, determining a display priority of each piece of the medical research information, and outputting a plurality of pieces of the medical research information based on display priorities, wherein the medical research information related to the abnormal physiological indicator of the user has a highest display priority.

19. The method according to any one of claims 7 to 18, further comprising:
receiving improvement suggestion information sent by the third device; and
outputting the prediction result and the improvement suggestion information.

20. A health management method, applied to a second device, wherein the method comprises:
determining a target physiological indicator of a user in response to a selection operation of the user;
sending the target physiological indicator of the user to a first device; and
obtaining living data information and physiological data information of the user, and sending the living data information and the physiological data information of the user to the first device.

21. The method according to claim 20, wherein the obtaining living data information of the user comprises:
obtaining the living data information of the user in response to an input operation of the living data information of the user.

22. The method according to claim 20, wherein the obtaining physiological data information of the user comprises:
obtaining, from a fourth device, the physiological data information of the user monitored by the fourth device.

23. The method according to claim 20, further comprising:
in response to a physical examination information input operation of the user, performing recognition processing on physical examination information input by the user, to obtain physiological indicator data information of the user;
sending the physiological indicator data information of the user to the first device;
receiving abnormal physiological indicator information and normal physiological indicator information of the user sent by the first device; and
outputting the abnormal physiological indicator information and the normal physiological indicator information of the user.

24. The method according to claim 23, wherein the method further comprises:
detecting whether the user has a physiological data detection device, wherein the physiological data detection device is a device for detecting at least one physiological indicator of the user; and
when it is detected that the user has the physiological data detection device, displaying the abnormal physiological indicator information in a preset sequence; and
the determining a target physiological indicator of a user in response to a selection operation of the user comprises:
determining the target physiological indicator of the user in response to the selection operation of the user on the abnormal physiological indicator information in the abnormal physiological indicator information displayed in the preset sequence.

25. The method according to claim 24, further comprising:
when it is detected that the user does not have the physiological data detection device, outputting information about a physiological data detection device capable of detecting an abnormal physiological indicator of the user.

26. The method according to claim 24, wherein the method further comprises:
detecting whether the physiological data detection device of the user comprises a fourth device, wherein the fourth device is a physiological data detection device capable of detecting the target physiological indicator of the user; and
the sending the target physiological indicator of the user to a first device comprises:
when the physiological data detection device of the user comprises the fourth device, sending the target physiological indicator of the user to the first device.

27. The method according to claim 26, further comprising:
when it is detected that the physiological data detection device of the user does not comprise the fourth device, outputting information about the fourth device.

28. The method according to any one of claims 24 to 27, wherein before the detecting whether the user has a physiological data detection device, the method further comprises:
generating recommendation information based on the abnormal physiological indicator information of the user and displaying the recommendation information, wherein the recommendation information comprises at least one of the information about the physiological data detection device capable of detecting the abnormal physiological indicator of the user and medical research information related to the abnormal physiological indicator of the user; and
the detecting whether the user has a physiological data detection device comprises:
in response to a first operation of the user on the recommendation information, detecting whether the user has the physiological data detection device.

29. The method according to claim 28, further comprising:
outputting information about the abnormal physiological indicator of the user in response to a second operation of the user on the recommendation information; or
in response to a third operation of the user on the recommendation information, outputting the information about the physiological data detection device capable of detecting the abnormal physiological indicator of the user; or
outputting the medical research information related to the abnormal physiological indicator of the user in response to a fourth operation of the user on the recommendation information.

30. The method according to any one of claims 23 to 29, further comprising:
in response to a display operation of the user for medical research information, determining a display priority of each piece of the medical research information, and outputting a plurality of pieces of the medical research information based on display priorities, wherein the medical research information related to the abnormal physiological indicator of the user has a highest display priority.

31. The method according to any one of claims 20 to 30, further comprising:
obtaining improvement suggestion information for the target physiological indicator and a prediction result; and
outputting the prediction result and the improvement suggestion information.

32. A health management apparatus, comprising:
an obtaining unit, configured to determine a target physiological indicator of a user; and
a processing unit, configured to obtain living data information and physiological data information of the user, and input the living data information and the physiological data information of the user into a prediction model, to obtain a prediction result output by the prediction model, wherein the prediction result represents a predicted impact of the living data information and the physiological data information of the user on a change trend of the target physiological indicator.

33. A health management apparatus, comprising:
an obtaining unit, configured to determine a target physiological indicator of a user in response to a selection operation of the user; and
a sending unit, configured to send the target physiological indicator of the user to a first device, wherein
the obtaining unit is further configured to obtain living data information and physiological data information of the user; and
the sending unit is further configured to send the living data information and the physiological data information of the user to the first device.

34. An electronic device, comprising a memory configured to store computer program instructions and a processor configured to execute the program instructions, wherein when the computer program instructions are executed by the processor, the electronic device is triggered to perform the method according to any one of claims 1 to 19 or the method according to any one of claims 20 to 31.

35. A computer-readable storage medium, wherein the computer-readable storage medium comprises a stored program, and when the program is run, a device in which the computer-readable storage medium is located is controlled to perform the method according to any one of claims 1 to 19 or the method according to any one of claims 20 to 31.
